# EUROPEAN PATENT APPLICATION

(11) **EP 4 751 777 A1**
(43) Date of publication of application: **03.06.2026**
(21) Application number: 24216653.6
(22) Date of filing: 29.11.2024
(51) Int. Cl.: A61P 35/00, C07K 16/28, A61K 39/00

(54) **COMBINATION OF A BISPECIFIC ANTIBODY CONSTRUCT, AN IMMUNE CHECKPOINT INHIBITOR AND A VEGFR-2 INHIBITOR**

(71) Applicant: Affimed GmbH, 68165 Mannheim (DE)
(72) Inventor: Pahl, Jens, 68165 Mannheim (DE); Stäble, Sina, 68165 Mannheim (DE); Morales-Espinosa, Daniela, 68165 Mannheim (DE); Dulat, Holger, 68165 Mannheim (DE); Schlenzka, Maria, 68165 Mannheim (DE)
(74) Representative: Schiweck Weinzierl Koch Patentanwälte Partnerschaft mbB

(57) **Abstract**

The present invention relates to a bispecific antibody construct and/or a VEGFR-2 inhibitor and/or an immune checkpoint inhibitor for use in a method of treating a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct, the VEGFR-2 inhibitor, and the immune checkpoint inhibitor. The invention further relates to corresponding (pharmaceutical) compositions and combination comprising the bispecific antibody construct and/or a VEGFR-2 inhibitor and/or an immune checkpoint inhibitor.

## Description

### Field of the invention

The present invention relates to a bispecific antibody construct and/or a VEGFR-2 inhibitor and/or an immune checkpoint inhibitor for use in a method of treating a tumor in a subject, wherein the bispecific antibody construct comprises at least one first binding domain capable of specifically binding CD16A and at least one second binding domain capable of specifically binding EGFR. The invention further relates to a (pharmaceutical) composition comprising a bispecific antibody construct that comprises at least one first binding domain capable of specifically binding CD16A and at least one second binding domain capable of specifically binding EGFR, as well as to a combination comprising a bispecific antibody construct that comprises at least one first binding domain capable of specifically binding CD16A and at least one second binding domain capable of specifically binding EGFR, and optionally an immune checkpoint inhibitor. The (pharmaceutical) composition and the combination as provided can be used in a method of treating a tumor in a subject.

### Background

The treatment of non-small cell lung cancer (NSCLC) in the second and third lines is critical due to several limitations of current therapies. In particular traditional chemotherapy agents like docetaxel and pemetrexed have shown limited efficacy in improving overall survival and quality of life for patients in the second and third lines of treatment (Morabito BMC Medicine (2018) 16:24). The response rates are generally low, and the duration of response is often short. Moreover, many of the currently used chemotherapies are associated with significant toxicity and adverse effects, which can severely impact the patient's quality of life (Favaretto, Europ Oncol (2009) 5(1):38). This is particularly challenging for patients who have already undergone first-line treatments and may have compromised health. Another issue is that tumors often develop resistance to initial treatments, making subsequent therapies less effective (Davies 2017,PLOS one 0175679). This resistance can be due to various genetic and molecular changes in the tumor cells, which necessitates the need for new treatments that can overcome or bypass these resistance mechanisms. While newer therapies such as immunotherapy (e.g., nivolumab, pembrolizumab) and targeted therapies (e.g., EGFR inhibitors) have shown promise, they are not effective for all patients. There is a significant portion of patients who do not respond to these treatments or who experience progression after an initial response. Given these challenges, there is a high medical need for the development of new and more effective treatments for NSCLC patients in the second and third lines of therapy. These treatments should ideally have better efficacy, lower toxicity, and the ability to overcome resistance mechanisms.

Previously, it has been shown in experimental mouse models and in clinical studies that anti-VEGF receptor antibodies can reshape the tumor microenvironment towards a less immunosuppressive milieu, including reduction in Treg cells in tumor-infiltrating lymphocytes (Terme et al. Cancer Res . 2013 Jan 15;73(2):539-49; Tada et al. J Immunother Cancer 2018 Oct 11;6(1): 106; Ziogas et al. Int J Cancer . 2012 Feb 15;130(4):857-64; ). In addition anti-VEGF receptor antibodies promoting normalization of the tumor vasculature and reduced hypoxia, supporting anti-tumor immune responses (Huang et al. Proc Natl Acad Sci U S A . 2012 Oct 23; 109(43): 17561-6); However, at the same time, anti-VEGF receptor antibodies can also induce potentially disadvantageous properties such as up-regulation of PD-L1 (Tada et al. J Immunother Cancer 2018 Oct 11;6(1):106). High doses of anti-VEGF receptor antibodies unexpectedly failed to improve normalization of the tumor vasculature in experimental mouse models (Huang et al. Proc Natl Acad Sci U S A . 2012 Oct 23; 109(43): 17561-6). Overall, so far it is unknown whether anti-VEGF receptor antibodies can act in synergy with NK cell-based immunotherapy.

In view of the existing need in the art for the provision of improved therapies comprising use of a bispecific antibody construct comprising at least one first binding domain capable of specifically binding CD16A and at least one second binding domain capable of specifically binding EGFR for treating cancerous diseases. The present invention addresses this need as indicated herein.

### Summary

The inventors observed that in the course of administration of a bispecific antibody construct comprising at least one first binding domain capable of specifically binding CD16A and at least one second binding domain capable of specifically binding EGFR, the number of Treg cells in the tumor or in the tumor environment increased together with an increased gene expression of TGF-β and the VEGF signaling pathway. However, an increase in Treg cells, TGF-β and VEGF signaling shapes the tumor microenvironment rather to an immunosuppressive milieu that curtails anti-tumor immunity while promoting tumor progression. Hence, such an immunosuppressive character of the tumor microenvironment is undesirable because it reduces the anti-tumor activity of NK cells and T cells including response to PD-1/PD-L1 immune checkpoint blockade. Thus, to maximize the outcome of NK cell-based immunotherapy, immunosuppression in the tumor microenvironment needs to be overcome.

The inventors used a VEGF-R2 antagonist, e.g. antibodies, and observed that the number of Treg cells and/or TGF-β gene expression decreased. In other words, with the administration of a VEGF-R2 antagonist, the inventors were able to counteract the unexpected finding that the administration of a bispecific antibody construct comprising at least one first binding domain capable of specifically binding CD16A and at least one second binding domain capable of specifically binding EGFR causes an increase in the number of Treg cells and TGF-β gene expression , so that the increase in immunosuppressive Treg cells and/or TGF-β gene expression decreased. In addition to a bispecific antibody construct comprising at least one first binding domain capable of specifically binding CD16A and at least one second binding domain capable of specifically binding EGFR, and a VEGF-R2 antagonist, a PD-L1 antagonist, e.g. an antibody, can also be administered. It has been observed that a bispecific antibody construct as herein described works synergistically with PD-L1 antagonists [PCT/IB2024/055675].

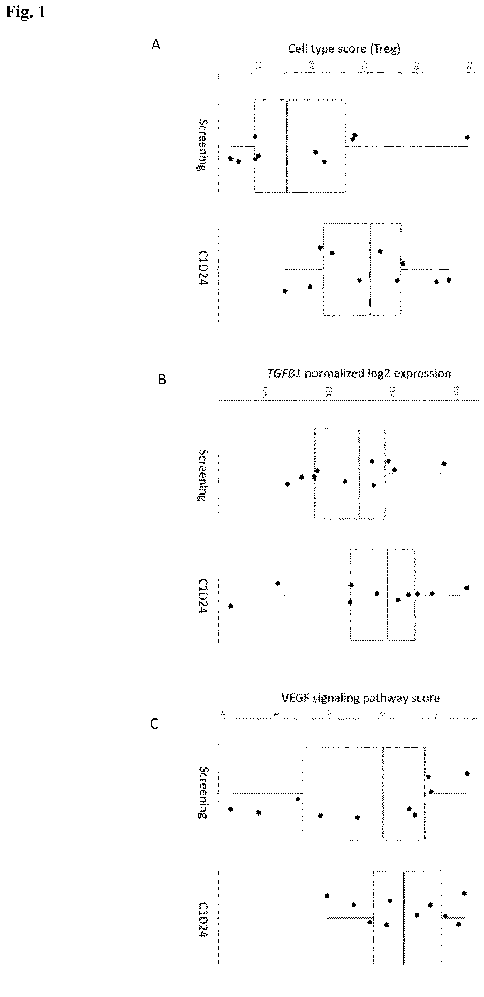

Thus, the present invention is based at least partly on the surprising finding that an increase in the number of regulatory T cells (Treg cells) is unexpectedly induced by the use of a bispecific antibody construct comprising at least one first binding domain capable of specifically binding CD16A and at least one second binding domain capable of specifically binding EGFR in a method of treating a tumor in a subject. The inventors found that in these subjects (patients), the increase in the number of Treg cells can be counteracted by the administration of a VEGF-R2 antagonist and an immune checkpoint inhibitor, so that the increase in immunosuppressive Treg cells is decreased. Specifically, by using FOXP3 as a specific marker gene for Tregs for calculating a Treg-specific cell type score, the inventors of the present invention surprisingly observed, that the amount of Treg cells is increased in patients upon AFM24 treatment **(****Figure 1** **A**). Thus, the data received by the inventors of the present invention demonstrates that a VEGFR-2 inhibitor and an immune checkpoint inhibitor are particularly useful in combination with a bispecific antibody construct, for treating a tumor in a subject.

In sum, the data provided herein indicates that the combination of a bispecific antibody construct as herein described, a VEGFR-2 inhibitor as herein described and an immune checkpoint inhibitor as herein described can be successfully used to treat a tumor in subjects.

Accordingly, in one aspect the present invention provides for a bispecific antibody construct and/or a VEGFR-2 inhibitor and/or an immune checkpoint inhibitor for use in a method of treating a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct, the VEGFR-2 inhibitor, and the immune checkpoint inhibitor.

In some cases, the tumor may be a solid tumor and/or the tumor may be characterized by being an EGFR-positive tumor, and or by EGFR overexpression on tumor cells.

In some embodiments, the first binding domain binds to an epitope on CD16A which is C-terminal to the physiological Fcy receptor binding domain, said epitope preferably comprising Y158 of SEQ ID NO: 1.

In some embodiments, the first binding domain comprises
a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13);
a VH region comprising CDR-H1 sequence GYTFTSYY (SEQ ID NO: 17), CDR-H2 sequence IEPMYGST (SEQ ID NO: 18), and CDR-H3 sequence ARGSAYYYDFADY (SEQ ID NO: 19), and a VL region comprising CDR-L1 sequence NIGSKN (SEQ ID NO: 20), CDR-L2 sequence QDN, and CDR-L3 sequence QVWDNYSVL (SEQ ID NO: 21);
a VH region comprising CDR-H1 sequence GYTFTNYY (SEQ ID NO: 25), CDR-H2 sequence INPSGGVT (SEQ ID NO: 26), and CDR-H3 sequence ARGSAYYYDFADY (SEQ ID NO: 27), and a VL region comprising CDR-L1 sequence NIGSKS (SEQ ID NO: 28), CDR-L2 sequence QDK, and CDR-L3 sequence QVWDDYIVL (SEQ ID NO: 29);
a VH region comprising CDR-H1 sequence SYYMH (SEQ ID NO: 32), CDR-H2 sequence AIEPRYGSTSYAQKFQG (SEQ ID NO: 33), and CDR-H3 sequence GSAYYYDFADY (SEQ ID NO: 34), and a VL region comprising CDR-L1 sequence GGHNIGSKNVH (SEQ ID NO: 35), CDR-L2 sequence QDNKRPS (SEQ ID NO: 36), and CDR-L3 sequence QVWDNYNVL (SEQ ID NO: 37); or
a VH region comprising CDR-H1 sequence NYYMQ (SEQ ID NO: 38), CDR-H2 sequence IINPSGGVTSYAQKFQG (SEQ ID NO: 39), and CDR-H3 sequence GSAYYYDFADY (SEQ ID NO: 40), and a VL region comprising CDR-L1 sequence GGNNIGSKSVH (SEQ ID NO: 41), CDR-L2 sequence QDKKRPS (SEQ ID NO: 42), and a CDR-L3 sequence QVWDDYIVL (SEQ ID NO: 43).

In some embodiments, the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).

In some embodiments, the second binding domain of the bispecific antibody construct is a variable domain (Fv), a single chain Fv (scFv), a Fab, a single chain diabody (scDb), a diabody (Db) or a double Fab, preferably a double Fab.

In some embodiments, the bispecific antibody construct binds to a target cell and an immune effector cell simultaneously.

In some embodiments, the bispecific antibody construct is AFM24.

In some embodiments, the at least one first binding domain of the bispecific antibody construct comprises means for specifically binding CD16A and the at least one second binding domain comprises means for specifically binding EGFR. In some embodiments, the bispecific antibody construct comprises means for specifically binding CD16A and EGFR.

In some embodiments, the bispecific antibody construct is administered at a dose in the range of about 300 to about 800 mg, about 400 to about 550 mg, about 420 to about 530 mg, about 450 to about 500 mg, or about 480 mg. In some embodiments, the VEGFR-2 inhibitor is administered at a dose in the range of about 5 to about 15 mg, about 8 to about 12 mg, about 9 to about 11 mg, or about 10 mg. In some embodiments, the immune checkpoint inhibitor is administered at a dose in the range of about 500 to about 1200 mg, about 700 to about 1000 mg, about 800 to about 900 mg, or about 840 mg.

According to a further aspect, the present invention provides a (pharmaceutical) composition comprising a VEGFR-2 inhibitor and a bispecific antibody construct that comprises at least one first binding domain capable of specifically binding CD16A and at least one second binding domain capable of specifically binding EGFR, and optionally an immune checkpoint inhibitor.

In some embodiments, the VEGFR-2 inhibitor is present in an amount (by weight) that is in the range of about 40 to about 60 times lower than an amount of the bispecific antibody construct.

According to a further aspect, the present invention provides a combination comprising a VEGFR-2 inhibitor, a bispecific antibody construct that comprises at least one first binding domain capable of specifically binding CD16A and at least one second binding domain capable of specifically binding EGFR, and optionally an immune checkpoint.inhibitor.

In some embodiments, the combination is for use in a method of treating a tumor in a subject.

According to a further aspect, the present invention provides a VEGFR-2 inhibitor or a combination of a VEGFR-2 inhibitor, a bispecific antibody construct and an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the increase of regulatory T cells in a tumor microenvironment associated with the treatment of a tumor with a bispecific antibody construct and optionally an immune checkpoint inhibitor.

According to a further aspect, the present invention provides a VEGFR-2 inhibitor or a combination of a VEGFR-2 inhibitor, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the transformation of a tumor microenvironment to an immune-suppressive tumor-microenvironment associated with the treatment of a tumor with a bispecific antibody construct and optionally an immune checkpoint inhibitor.

According to a still further aspect, the present invention provides a VEGFR-2 inhibitor or a combination of a VEGFR-2 inhibitor, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting neovascularization associated with the treatment of a tumor with a bispecific antibody construct and optionally an immune checkpoint inhibitor.

According to a further aspect, there is provided a method of treating a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct and the VEGFR-2 inhibitor.

In some embodiments, the (pharmaceutical) compositions and combinations as herein disclosed are used as defined in any of the methods described herein.

Also provided is a use of a bispecific antibody construct and/or a VEGFR-2 inhibitor and/or an immune checkpoint inhibitor for the manufacture of a medicament for treatment of a a tumor in a subject, wherein the bispecific antibody construct comprises at least one first binding domain capable of specifically binding CD16A and at least one second binding domain capable of specifically binding EGFR.

### Brief description of the drawings

**Figure 1: Fig. 1** shows Gene expression profiling of tissue biopsies indicating the induction of an immune suppressive tumor microenvironment upon AFM24 treatment, wherein **Fig. 1A** shows results of a Cell type score calculation for Tregs based on FOXP3 expression in paired biopsies from screening and at C1D24 of patients treated with AFM24 at doses higher than 160 mg, **Fig 1B** shows results of a quantification of TGFB 1 expression in paired biopsies from screening and at C1D24 of patients treated with AFM24 at doses higher than 160 mg; and **Fig 1C** shows results of a calculation of a pathway score for the VEGF signaling cascade in paired biopsies from screening and at C1D24 of patients treated with AFM24 at doses higher than 160 mg.

### Detailed description

This invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

Based on the data provided in the present application the inventors of the present invention believe that the combined use of a VEGFR-2 inhibitor, a bispecific antibody construct, specifically a bispecific antibody construct comprising at least one first binding domain capable of specifically binding CD16A and at least one second binding domain capable of specifically binding EGFR, and an immune checkpoint inhibitor is particularly beneficial for treating an EGFR-positive tumor disease in a patient. In particular, the data provided herein clearly demonstrate that the administration of a VEGFR-2 inhibitor and of an immune checkpoint inhibitor is particularly effective in combined treatment together with administration of a bispecific antibody construct comprising at least one first binding domain capable of specifically binding CD16A and at least one second binding domain capable of specifically binding EGFR as herein defined for treating a tumor in a subject. **(****Figures 1** **A, B and C).**

Accordingly, in accordance with one aspect of the present invention, there is provided a bispecific antibody construct and/or a VEGFR-2 inhibitor and or an immune checkpoint inhibitor for use in a method of treating a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct, the VEGFR-2 inhibitor, and an immune checkpoint inhibitor.

As used herein, the terms "treatment," "treat," and "treating" refer to reversing, alleviating, delaying the onset of, or inhibiting the progress of a tumor disease. For example, a treatment can result in a reduction in the amount of tumor cells or a reduced growth rate. Administration of a therapeutically effective amount of a compound described herein for the treatment of an EGFR-positive tumor disease will result in a reduction of the amount of tumor cells or a decreased growth rate, a reduction in risk or frequency of reoccurrence, a delay in reoccurrence, a reduction in metastasis, increased survival, and/or decreased morbidity and mortality, among other things. In some embodiments, treatment may be administered after one or more symptoms have developed. In other embodiments, treatment may be administered in the absence of symptoms. For example, a treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence.

Thus, also provided herein are methods of preventing, reducing and/or inhibiting the recurrence, growth, proliferation, migration and/or metastasis of a cancer cell or population of cancer cells in a subject suffering from a tumor disease, comprising administering to the subject the bispecific antibody construct, the VEGFR-2 inhibitor, and the immune checkpoint inhibitor as described herein.

Also provided herein are methods of enhancing, improving, and/or increasing the response to an anticancer therapy in a subject suffering from a tumor disease, in particular a tumor disease being EGFR positive or being characterized by epidermal growth factor receptor (EGFR) overexpression on tumor cells, comprising administering to said patient the bispecific antibody construct, the VEGFR-2 inhibitor, and the immune checkpoint inhibitor as described herein.

As used herein, the term "inhibition", as it relates to cancer and/or cancer cell proliferation, refers to the inhibition of the growth, division, maturation or viability of cancer cells, and/or causing the death of cancer cells, individually or in aggregate with other cancer cells, by cytotoxicity, nutrient depletion, or the induction of apoptosis.

As used herein, "delaying" the onset or "delaying" reoccurrence of a disease or disorder, or one or more symptoms thereof, means to defer, hinder, slow, retard, stabilize and/or postpone development of the disease, disorder, or symptom thereof. This delay can be of varying lengths of time, depending on the history of the disease and/or subject being treated. As it is evident to one skilled in the art, a sufficient or significant delay can, in effect, encompass prevention, in that the subject does not develop the disease, disorder, or symptom thereof. For example, a method that "delays" development of cancer or reoccurrence of the cancer is a method that reduces the probability of disease development in a given time frame and/or reduces extent of the disease in a given time frame, when compared to not using the described method. Such comparisons may be based on clinical studies, using a statistically significant number of subjects.

As used herein, "prevention" or "preventing" refers to a regimen that protects against the onset of the disease or disorder such that the clinical symptoms of the disease do not develop. Thus, "prevention" relates to administration of a therapy (e.g., administration of a therapeutic substance) to a subject before signs of the disease are detectable in the subject and/or before a certain stage of the disease (e.g., administration of a therapeutic substance to a subject with a cancer that has not yet metastasized). The subject may be an individual at risk of developing the disease or disorder, or at risk of disease progression, e.g., cancer metastasis. Such as an individual who has one or more risk factors known to be associated with development or onset of the disease or disorder. For example, an individual may have mutations associated with the development or progression of a cancer. Further, it is understood that prevention may not result in complete protection against onset of the disease or disorder. In some instances, prevention includes reducing the risk of developing the disease or disorder. The reduction of the risk may not result in complete elimination of the risk of developing the disease or disorder.

As set forth above, the present invention also relates to a bispecific antibody construct and/or a VEGFR-2 inhibitor and/or an immune checkpoint inhibitor for use in a method of treating a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct, the VEGFR-2 inhibitor, and an immune checkpoint inhibitor.

Patients that can be treated using the means and methods described herein include those subjects that have undergone "previous treatment". "Previous treatment" as used herein refers to treatment of the tumor disease that occurs prior to the treatment provided by the present invention, i.e. prior to administering a combination of the bispecific antibody construct, the VEGFR-2 inhibitor, and the immune checkpoint inhibitor to said patient as herein described.

The subject may be undergoing one or more standard therapies for treating tumor disease, such as chemotherapy, radiotherapy, immunotherapy, stem cell therapy or combinations thereof. For example, the previous treatment of the tumor disease may comprise traditional chemotherapy (e.g. treatment with cisplatin, docetaxel, carboplatin, gemcitabine, cisplatin, pemetrexed, or combinations thereof). Patients previously treated with platinum-based drugs may have had their treatment discontinued due to systemic toxicity and drug resistance. In some embodiments, previous treatment of the tumor disease may comprise treatment with cytarabine or an anthracycline, such as daunorubicin or idarubicin.

In some embodiments, the subject may also be one that shows satisfactory results at the onset of an initial treatment, but becomes less responsive to said treatment after a period of time. Subjects having received previous treatments may have undergone treatments with different drugs, or drug combinations, at different times. For example, some subjects may have undergone two or more therapies using different drugs of drug combinations, wherein the therapies were discontinued as not providing desired results.

The terms "subject," "individual," or "patient" are often used interchangeably herein. Subjects "suffering from a disease" or those "in need of treatment" include those already with the disorder or disease, as well as those in which onset or reoccurrence of the disorder or disease, or symptoms thereof as described elsewhere herein, is to be prevented or delayed. Suitable patients for the means and methods described herein include those who are suffering from, who have been diagnosed with, or who are suspected of having a tumor disease.

The term *"in vivo"* is used to describe an event that takes place in a subject's body. The term *"ex vivo"* is used to describe an event that takes place outside of a subject's body. An *ex vivo* assay is not performed on a subject. Rather, it is performed upon a sample separate from a subject. An example of an ex vivo assay performed on a sample is an *"in vitro"* assay. The term *"in vitro"* is used to describe an event that takes places contained in a container for holding laboratory reagent such that it is separated from the biological source from which the material is obtained. *In vitro* assays can encompass cell-based assays in which living or dead cells are employed. *In vitro* assays can also encompass a cell-free assay in which no intact cells are employed.

In some embodiments, the subject is a mammal selected from the group consisting of an armadillo, an ass, a bat, a bear, a beaver, a cat, a chimpanzee, a cow, a coyote, a deer, a dog, a dolphin, an elephant, a fox, a panda, a gibbon, a giraffe, a goat, a gopher, a hedgehog, a hippopotamus, a horse, a humpback whale, a jaguar, a kangaroo, a koala, a leopard, a lion, a llama, a lynx, a mole, a monkey, a mouse, a narwhal, an orangutan, an orca, an otter, an ox, a pig, a polar bear, a porcupine, a puma, a rabbit, a raccoon, a rat, a rhinoceros, a sheep, a squirrel, a tiger, a walrus, a weasel, a wolf, a zebra, a goat, a horse, and combinations thereof. In some embodiments, the mammal is a human. Thus, subjects to be treated using the means and methods provided herein include human and other mammalian subjects, who have been diagnosed with or are suspected of having a tumor disease. In some embodiments, the subject may be a human who exhibits one or more symptoms associated with a tumor disease.

The subject, e.g., the human subject, can be a child, e.g., from or from about 0 to or to about 14 years in age. The subject can be a youth, e.g., from or from about 15 to or to about 24 years in age. The subject can be an adult, e.g., from or from about 25 to or to about 64 years in age. The subject can be a senior, e.g. 65+ years in age.

Any of the means and methods of treatment provided herein may be used to treat a tumor disease at various stages. By way of example, the cancer stage includes but is not limited to early, advanced, locally advanced, remission, refractory, reoccurred after remission and progressive. In some embodiments, the subject is at an early stage of a cancer. In other embodiments, the subject is at an advanced stage of cancer. In various embodiments, the subject has a stage I, stage II, stage III or stage IV cancer. The means and methods described herein can promote reduction or retraction of a tumorous cells, decrease or inhibit cancer cell proliferation, and/or induce, increase or promote tumor cell killing. In some embodiments, the subject is in cancer remission. The means and methods described herein can prevent or delay metastasis or recurrence of cancer.

In certain embodiments, the subject may be a human who is (i) substantially refractory to at least one chemotherapy treatment, or (ii) is in relapse after treatment with chemotherapy, or both (i) and (ii). In some of embodiments, the subject is refractory to at least two, at least three, or at least four chemotherapy treatments (including standard or experimental chemotherapies described elsewhere herein).

In some embodiments, the subject has relapsed after treatment with or is refractory to an antibody therapy as described e.g. in Morse at al. 2023, Expert Opinion on Investigational Drugs, 32 (2): 107-125. In some embodiments, the subject is refractory to or has a recurrence after treatment with a tyrosine kinase inhibitor. In some embodiments, the tyrosine kinase inhibitor is selected from the group consisting of gefitinib, erlontinib, afatinib, osimertinib, and combinations thereof.

In some embodiments, the tumor may be a solid tumor. For example, the tumor may be characterized by epidermal growth factor receptor (EGFR) overexpression on tumor cells.

According to an embodiment, the tumor may be an EGFR-positive tumor, e.g. such a tumor may comprise tumor cells which express or overexpress EGFR.

In accordance with some embodiments, the tumor may be selected from the group consisting of lung cancer, colorectal cancer, liver cancer, brain cancer, kidney/renal cancer, ovarian cancer, breast cancer, squamous cell carcinoma, bladder cancer, head and neck cancer, gastric cancer, esophagus cancer, sarcoma, mesothelioma, and adenocarcinoma, optionally, non-small cell lung cancer (NSCLC), hepatocellular carcinoma (HCC), glioblastoma (GBM), Clear Cell Renal Cell Carcinoma (ccRCC), Triple-negative breast cancer (TNBC), squamous cervical cancer, and squamous cell carcinoma of head and neck (SCCHN).

In accordance with some embodiments, the tumor may be lung cancer, such as non-small cell lung cancer (NSCLC).

The tumor has a surrounding named tumor microenvironment comprising cancer cells, stromal tissue, extracellular matrix, and possibly blood vessels within and surrounding the tumor tissue. The tumor microenvironment comprises tumor-associated macrophages and T cells. Due to the immunosuppressive characteristics of the tumor microenvironment, for example by expression of checkpoint inhibitors, some macrophages may be M2 polarized and anti-inflammatory, thus tumor-promoting. Furthermore, T cells within the immunosuppressive tumor environment may be partly turned to regulatory T cells (Treg cells), which are tumor promoting and immunosuppressive by e.g. making CD8+ cell less effective.

In accordance with some embodiments, the first binding domain of the bispecific antibody construct binds to an epitope on CD16A which is C-terminal to the physiological Fcy receptor binding domain, said epitope preferably comprising Y158 of SEQ ID NO: 1.

As used herein, the term "bispecific antibody construct" refers to an antibody construct which is bispecific, i.e., it comprises at least a first binding domain and a second binding domain, wherein the first binding domain binds to one antigen or target (here: NK cell receptor CD16A), and the second binding domain binds to another antigen or target (here: the target cell surface antigen EGFR).

Examples of bispecific antibody constructs are provided, for example, in WO 2006/125668, WO 2017/064221, WO 2019/175368, WO 2019/198051, WO 2020/043670, WO 2021/130383, and Ellwanger et a. (MAbs. 2019 Jul;11(5):899-918).

A bispecific antibody or an antigen binding fragment thereof may comprise a first binding domain capable of specifically binding CD16A and a second binding domain capable of specifically binding EGFR. The bispecific antibody construct disclosed herein may comprise a CD16A binding domain and a EGFR binding domain.

Given that the antibody constructs as defined in the context of the invention are (at least) bispecific, they do not occur naturally and they are markedly different from naturally occurring products. A "bispecific" antibody construct or immunoglobulin is hence an artificial hybrid antibody or immunoglobulin having at least two distinct binding sides with different specificities. Bispecific antibody constructs can be produced by a variety of methods including fusion of hybridomas or linking of Fab' fragments. See, e.g., Songsivilai & Lachmann, Clin. Exp. lmmunol. 79:315- 321 (1990).

As used herein, "CD16A" refers to the activating receptor CD16A, also known as Fc□RIIIA, expressed on the cell surface ofNK cells. CD16A is an activating receptor triggering the cytotoxic activity ofNK cells. The affinity of antibodies for CD16A directly correlates with their ability to trigger NK cell activation, thus higher affinity towards CD16A reduces the antibody dose required for activation. The antigen-binding site of the antigen-binding protein binds to CD16A, but not to CD16B. For example, an antigen-binding site comprising heavy (VH) and light (VL) chain variable domains binding to CD16A, but not binding to CD16B, may be provided by an antigen binding site which specifically binds to an epitope of CD16A which comprises amino acid residues of the C-terminal sequence SFFPPGYQ (SEQ ID NO: 70) and/or residues Gl30 and/or Y141 of CD16A (SEA ID NO: 71) which are not present in CD16B. In some embodiments, the antigen-binding site for CD16A does not bind to CD16B and binds to the known CD16A allotypes F158 and V158 with similar affinity. Two allelic single nucleotide polymorphisms have been identified in human CD16A altering the amino acid in position 158, which is important for interaction with the hinge region of IgG. The allelic frequencies of the homozygous 158 F/F and the heterozygous 158 V/F alleles are similar within the Caucasian population, ranging between 35 and 52% or 38 and 50%, respectively, whereas the homozygous 158 V/V allele is only found in 10-15% (Lopez-Escamez JA et al .; BMC Med Genet 2011; 12: 2). Activation of NK-cells by this anti-CD16A domain in all patients due to the similar affinity is therefore advantageous. Further CD16A antigen-binding sites comprising heavy and light variable chain domains that bind to CD16A, but not to CD16B are described in WO 2006/125668.

The term "EGFR" refers to the epidermal growth factor receptor (EGFR; ErbB-1; HER1) in humans, including all isoforms or variants described with activation, mutations and implicated in pathophysiological processes. A binding domain specific for EGFR may recognize an epitope in the extracellular domain of the EGFR. A binding domain specific for EGFR may specifically bind to human and cynomolgus EGFR, i.e. the binding domain may be cross-reactive to human and cynomolgus EGFR. The epidermal growth factor receptor (EGFR) is a member of the HER family of receptor tyrosine kinases and consists of four members: EGFR (ErbB1/HER1), HER2/neu (ErbB2), HER3 (ErbB3) and HER4 (ErbB4). Stimulation of the receptor through ligand binding (e.g. EGF, TGFa, HB-EGF, neuregulins, betacellulin, amphiregulin) activates the intrinsic receptor tyrosine kinase in the intracellular domain through tyrosine phosphorylation and promotes receptor homo- or heterodimerization with HER family members. These intracellular phospho-tyrosines serve as docking sites for various adaptor proteins or enzymes including SHC, GRB2, PLCg and PI(3)K/Akt, which simultaneously initiate many signaling cascades that influence cell proliferation, angiogenesis, apoptosis resistance, invasion and metastasis. EGFR can be expressed by a number of different tumor types including lung (particularly non-small cell cancer NSCLC), colorectal (particularly CRC), liver (particularly hepatocellular carcinoma HCC), brain (particularly glioblastoma GBM), kidney/renal (particularly ccRCC), ovarian, breast (particularly TNBC), squamous cell carcinoma (particularly squamous cervical cancer), bladder, head and neck (particularly squamous cell carcinoma of head and neck SCCHN), gastric cancer, esophagus, sarcoma, mesothelioma, and adenocarcinoma.

The term "epitope" refers to a side on an antigen to which a binding domain, such as an antibody or immunoglobulin, or a derivative, fragment or variant of an antibody or an immunoglobulin, specifically binds. An "epitope" is antigenic and thus the term epitope is sometimes also referred to herein as "antigenic structure" or "antigenic determinant". Thus, the binding domain is an "antigen interaction site". Said binding/interaction is also understood to define a "specific recognition". "Epitopes" can be formed both by contiguous amino acids or non-contiguous amino acids juxtaposed by tertiary folding of a protein. A "linear epitope" is an epitope where an amino acid primary sequence comprises the recognized epitope. A linear epitope typically includes at least 3 or at least 4, and more usually, at least 5 or at least 6 or at least 7, for example, about 8 to about 10 amino acids in a unique sequence. A "conformational epitope", in contrast to a linear epitope, is an epitope wherein the primary sequence of the amino acids comprising the epitope is not the sole defining component of the epitope recognized (e.g., an epitope wherein the primary sequence of amino acids is not necessarily recognized by the binding domain). Typically, a conformational epitope comprises an increased number of amino acids relative to a linear epitope.

As used herein, "CD16B" refers to receptor CD16B, also known as FcyRIIIB, expressed on neutrophils and eosinophils. The receptor is glycosylphosphatidyl inositol (GPI) anchored and is understood to not trigger any kind of cytotoxic activity of CD16B positives immune cells.

In some embodiments, the first binding domain of the bispecific antibody construct used in the context of the means and methods of the present invention binds to an epitope on CD16A which is C-terminal to the physiological Fcy receptor binding domain, said epitope preferably comprising Y158 of SEQ ID NO: 1.

The term "binding domain" in connection with the present invention characterizes a domain which is capable of specifically binding to / interacting with / recognizing a given target epitope or a given target site on the target molecules (antigens), e.g. the NK cell receptor antigen CD16A on the surface of the NK cell, and a target cell surface antigen, i.e. EGFR on the tumor cell, respectively. Thus, in some embodiments, the bispecific antibody construct comprises at least a first binding domain capable of specifically binding CD16A on the surface of an immune effector and at least a second binding domain capable of specifically binding EGFR on the surface of a tumor cell.

The structure and/or function of the first binding domain (recognizing CD16A), and preferably also the structure and/or function of the second binding domain (recognizing the target cell surface antigen EGFR), is/are based on the structure and/or function of an antibody, e.g. of a full-length or whole immunoglobulin molecule and/or is/are drawn from the variable heavy chain (VH) and/or variable light chain (VL) domains of an antibody or fragment thereof. Preferably the first binding domain is characterized by the presence of three light chain CDRs (i.e. CDR1, CDR2 and CDR3 of the VL region) and/or three heavy chain CDRs (i.e. CDR1, CDR2 and CDR3 of the VH region). The second binding domain preferably also comprises the minimum structural requirements of an antibody which allow for the target binding. More preferably, the second binding domain comprises at least three light chain CDRs (i.e. CDRl, CDR2 and CDR3 of the VL region) and/or three heavy chain CDRs (i.e. CDR1, CDR2 and CDR3 of the VH region). In some cases, the first and/or second binding domain is produced by or obtainable by phage-display or library screening methods rather than by grafting CDR sequences from a pre-existing (monoclonal) antibody into a scaffold.

The term "variable" refers to the portions of the antibody or immunoglobulin domains that exhibit variability in their sequence and that are involved in determining the specificity and binding affinity of a particular antibody (i.e., the "variable domain(s)"). The pairing of a variable heavy chain (VH) and a variable light chain (VL) together forms a single antigen-binding side.

Variability is not evenly distributed throughout the variable domains of antibodies; it is concentrated in sub-domains of each of the heavy and light chain variable regions. These sub-domains are called "hypervariable regions" or "complementarity determining regions" (CDRs). The more conserved (i.e., non-hypervariable) portions of the variable domains are called the "framework" regions (FRM or FR) and provide a scaffold for the six CDRs in three-dimensional space to form an antigen-binding surface. The variable domains of naturally occurring heavy and light chains each comprise four FRM regions (FR1, FR2, FR3, and FR4), largely adopting a β-sheet configuration, connected by three hypervariable regions, which form loops connecting, and in some cases forming part of, the β-sheet structure. The hypervariable regions in each chain are held together in close proximity by the FRM and, with the hypervariable regions from the other chain, contribute to the formation of the antigen-binding side (see Kabat et al., loc. cit.).

The terms "CDR", and its plural "CDRs", refer to the complementarity determining region of which three make up the binding character of a light chain variable region (CDR-L1, CDR-L2 and CDR-L3) and three make up the binding character of a heavy chain variable region (CDR-H1, CDR-H2 and CDR-H3). CDRs contain most of the residues responsible for specific interactions of the antibody with the antigen and hence contribute to the functional activity of an antibody molecule: they are the main determinants of antigen specificity. The CDR3 of the light chain and, particularly, the CDR3 of the heavy chain may constitute the most important determinants in antigen binding within the light and heavy chain variable regions. In some antibody constructs, the heavy chain CDR3 appears to constitute the major area of contact between the antigen and the antibody. *In vitro* selection schemes in which CDR3 alone is varied can be used to vary the binding properties of an antibody or determine which residues contribute to the binding of an antigen. Hence, CDR3 is typically the greatest source of molecular diversity within the antibody-binding side. H3, for example, can be as short as two amino acid residues or greater than 26 amino acids.

The exact definitional CDR boundaries and lengths are subject to different classification and numbering systems. CDRs may therefore be referred to by IMGT, Kabat, Chothia, contact or any other boundary definitions, including the numbering system described herein. Despite differing boundaries, each of these systems has some degree of overlap in what constitutes the so called "hypervariable regions" within the variable sequences. CDR definitions according to these systems may therefore differ in length and boundary areas with respect to the adjacent framework region. See for example the IMGT method as described in Lefranc, M.-P., The Immunologist, 7, 132-136 (1999), Kabat (an approach based on cross-species sequence variability), Chothia (an approach based on crystallographic studies of antigen-antibody complexes), and/or MacCallum (Kabat et al., loc. cit; Chothia et al., J. Mol. Biol, 1987, 196: 901 -917; and MacCallum et al., J. Mol. Biol, 1996, 262: 732). Still another standard for characterizing the antigen binding side is the AbM definition used by Oxford Molecular's AbM antibody modeling software. See, e.g., Protein Sequence and Structure Analysis of Antibody Variable Domains. In: Antibody Engineering Lab Manual (Ed.: Duebel, S. and Kontermann, R., Springer-Verlag, Heidelberg). To the extent that two residue identification techniques define regions of overlapping, but not identical regions, they can be combined to define a hybrid CDR. However, the numbering in accordance with the so-called IMGT system or the Kabat system is preferred.

As used herein, the term "target cell surface antigen" refers to an antigenic structure expressed by a cell and which is present at the cell surface such that it is accessible for an antibody construct as described herein. In the context of the present invention, "target cell surface antigen" to which the bispecific antibody constructs described herein binds to is EGFR.

In some embodiments, binding domains are in the form of one or more polypeptides. Such polypeptides may include proteinaceous parts and non-proteinaceous parts (e.g. chemical linkers or chemical cross-linking agents such as glutaraldehyde). Proteins (including fragments thereof, preferably biologically active fragments, and peptides, usually having less than 30 amino acids) comprise two or more amino acids coupled to each other via a covalent peptide bond (resulting in a chain of amino acids).

In some embodiments, binding domains are in the form of one or more polypeptides. Such polypeptides may include proteinaceous parts and non-proteinaceous parts (e.g. chemical linkers or chemical cross-linking agents such as glutaraldehyde). Proteins (including fragments thereof, preferably biologically active fragments, and peptides, usually having less than 30 amino acids) comprise two or more amino acids coupled to each other via a covalent peptide bond (resulting in a chain of amino acids).

Preferably the binding domain which binds to the NK cell receptor antigen (CD16A) and/or the binding domain which binds to the target cell surface antigen EGFR is/are human, humanized or murine derived chimeric binding domains. Antibodies and antibody constructs comprising at least one human binding domain avoid some of the problems associated with antibodies or antibody constructs that possess non-human such as rodent (e.g. murine, rat, hamster or rabbit) variable and/or constant regions. The presence of such rodent derived proteins can lead to the rapid clearance of the antibodies or antibody constructs or can lead to the generation of an immune response against the antibody or antibody construct by a patient. In order to avoid the use of rodent derived antibodies or antibody constructs, human or fully human antibodies/ antibody constructs can be generated through the introduction of human antibody function into a rodent so that the rodent produces fully human antibodies.

In some embodiments, the at least one first binding domain described herein comprises means for specifically binding CD16A and the at least one second binding domain comprises means for specifically binding EGFR. In some embodiments the bispecific antibody construct comprises means for specifically binding CD16A and EGFR.

The term "specifically binding", as used herein means that the binding domain preferentially binds or recognizes the target even when the binding partner is present in a mixture of other molecules or other structures. The binding may be mediated by covalent or non-covalent interactions or a combination of both. In preferred examples, "simultaneous binding to a target cell and an immune effector cell" comprises the physical interaction between the binding domains and their targets on the cells, but preferably also includes the induction of an action mediated by the simultaneous binding of the two cells. Such an action may be an immune effector function of the immune effector cell, such as a cytotoxic effect. "Means" for specifically binding CD16A and EGFR are structures comprised by a binding domain that help to interact with the target molecule. Said structures are preferably one or more polypeptides described elsewhere herein.

The term "antibody construct" refers to a molecule in which the structure and/or function is/are based on the structure and/or function of an antibody, e.g., of a full-length or whole immunoglobulin molecule and/or is/are drawn from the variable heavy chain (VH) and/or variable light chain (VL) domains of an antibody or fragment thereof. An antibody construct is hence capable of binding to its specific target or antigen. Furthermore, the binding region of an antibody construct comprises the minimum structural requirements of an antibody which allow for the target binding. For the first binding domain to CD16A, this minimum requirement is defined by the presence of a VL region comprising the three light chain CDRs (i.e. CDR1, CDR2 and CDR3 of the VL region) and the presence of a VH region comprising the three heavy chain CDRs (i.e. CDR1, CDR2 and CDR3 of the VH region). For the second binding domain to a tumor antigen, this minimum requirement may, e.g., be defined by the presence of at least the three light chain CDRs (i.e. CDR1, CDR2 and CDR3 of the VL region) and/or the three heavy chain CDRs (i.e. CDR1, CDR2 and CDR3 of the VH region), preferably of all six CDRs. An alternative approach to define the minimal structure requirements of an antibody is the definition of the epitope of the antibody within the structure of the specific target, respectively, the protein domain of the target protein composing the epitope region (epitope cluster) or by reference to a specific antibody competing with the epitope of the defined antibody. The antibodies on which the constructs defined in the context of the invention are based include for example monoclonal, recombinant, chimeric, deimmunized, humanized and human antibodies.

The first binding domain of an antibody construct comprises the designated groups of CDRs. Those CDRs can be comprised in the framework of an antibody light chain variable region (VL) and an antibody heavy chain variable region (VH). The second binding domain comprises defined groups of CDRs. Preferably, those CDRs are comprised in the framework of an antibody light chain variable region (VL) and an antibody heavy chain variable region (VH); however, it does not have to comprise both. Fd fragments, for example, have two VH regions and often retain some antigen-binding function of the intact antigen-binding region.

Examples for the format of antibody fragments, antibody variants or binding domains include (1) a Fab fragment, a monovalent fragment having the VL, VH, CL and CH1 domains; (2) a F(ab')₂fragment, a bivalent fragment having two Fab fragments linked by a disulfide bridge at the hinge domain; (3) an Fd fragment having the two VH and CH1 domains; (4) an Fv fragment having the VL and VH domains of a single arm of an antibody, (5) a dAb fragment (Ward et al., (1989) Nature 341 :544-546), which has a VH domain; (6) an isolated complementarity determining region (CDR), and (7) a single chain Fv (scFv), the latter being preferred (for example, derived from an scFv-library). Examples for antibody constructs are e.g. described in WO00/006605, WO2005/040220, WO2008/119567, WO2010/037838, WO2013/026837, WO2013/026833, US2014/0308285, US2014/0302037, WO2014/144722, WO2014/151910, and WO2015/048272.

An antibody construct that can be used in methods of the disclosure can comprise a fragment of a full-length antibody, such as VH, VHH, VL, (s)dAb, Fv, Fd, Fab, Fab', F(ab')₂ or "r IgG" ("half antibody"). Antibody constructs can also comprise modified fragments of antibodies, also called antibody variants, such as scFv, di-scFv or bi(s)-scFv, scFv-Fc, scFv-zipper, scFab, Fab₂, Fabs, diabodies, single chain diabodies, tandem diabodies (Tandab's), tandem di-scFv, tandem tri-scFv, "multibodies" such as triabodies or tetrabodies, and single domain antibodies such as nanobodies or single variable domain antibodies comprising merely one variable domain, which might be VHH, VH or VL, that specifically bind an antigen or epitope independently of other V regions or domains.

As used herein, the terms "single-chain Fv," "single-chain antibodies" or "scFv" refer to single polypeptide chain antibody fragments that comprise the variable regions from both the heavy and light chains, but lack the constant regions. Generally, a single-chain antibody further comprises a polypeptide linker between the VH and VL domains which enables it to form the desired structure which would allow for antigen binding. Exemplary linkers for this purpose include glycine serine linkers, which preferably comprises from about 15 to about 30 amino acids. Preferred glycine serine linkers may have one or more repeats of GGS, GGGS (SEQ ID NO: 72), or GGGGS (SEQ ID NO: 5). Such linker preferably comprises 5, 6, 7, 8, 9 and/or 10 repeats of GGS, preferably (GGS)₆ (SEQ ID NO:2, which are preferably used for scFvs having the arrangement VH-VL), or preferably (GGS)₇ (SEQ ID NO: 3, which are preferably used for scFvs having the arrangement VL-VH). Single chain antibodies are discussed in detail by Plueckthun in The Pharmacology of Monoclonal Antibodies, vol. 1 13, Rosenburg and Moore eds. Springer-Verlag, New York, pp. 269-315 (1994). Various methods of generating single chain antibodies are known, including those described in U.S. Pat. Nos. 4,694,778 and 5,260,203; International Patent Application Publication No. WO 88/01649; Bird (1988) Science 242:423-442; Huston et al. (1988) Proc. Natl. Acad. Sci. USA 85:5879-5883; Ward et al. (1989) Nature 334:54454; Skerra et al. (1988) Science 242:1038- 1041. In specific embodiments, single-chain antibodies can also be human, and/or humanized and/or synthetic. The term "bi-scFv" or "ta-scFv" (tandem scFv) as used herein refers to two scFv that are fused together. Such a bi-scFv or ta-scFv may comprise a linker between the two scFv moieties. Generally, the arrangement of the VH and VL domains on the polypeptide chain within each of the scFv may be in any order. This means that the "bi-scFv" of "ta-scFv" can be arranged in the order VH(1)-VL(1)-VH(2)-VL(2), VL(1)-VH(1)-VH(2)-VL(2), VH(1)-VL(1)-VL(2)-VH(2), or VL(1)-VH(1)-VL(2)-VH(2), where (1) and (2) stand for the first and second scFv, respectively. The term "double Fab" as used herein refers to two Fab fragments that are fused together, which are preferably staggered. Here, a first chain of a first Fab is N-terminally fused to a first chain of a second Fab, or a second chain of a first Fab is N-terminally fused to a second chain of a second Fab, or both, the first chain of a first Fab and the second chain of a first Fab are fused to first and second chains of a second Fab, respectively. A linker may be present between the fused chains of the first and second Fab. The first and second chains of the first and second Fab can be individually selected from a light chain-derived chain of a Fab (VL-CL), a heavy chain derived chain of a Fab (VH-CH1), as long as each Fab contains a VH, a VL, a CH1, and a CL. As an illustrative example, the light chain-derived chain of the first Fab can be fused to the light chain derived-chain of the second Fab. As another illustrative example, the heavy chain-derived chain of the first Fab can be fused to the heavy chain derived-chain of the second Fab. As a further illustrative example, the heavy chain-derived chain of the first Fab can be fused to the light chain derived-chain of the second Fab. In some double Fabs, both chains of the two Fabs are fused together. For example, the light chain-derived chain of the first Fab can be fused to the light chain derived-chain of the second Fab while the heavy chain-derived chain of the first Fab can be fused to the heavy chain derived-chain of the second Fab. Alternatively, the light chain-derived chain of the first Fab can be fused to the heavy chain derived-chain of the second Fab while the heavy chain-derived chain of the first Fab can be fused to the light chain derived-chain of the second Fab.

In some embodiments, the binding domain specifically binding CD16A of the bispecific antibody construct comprises a heavy chain variable domain (VH_CD16a) comprising a heavy chain complementarity determining region 1 (CDR-H1) having the sequence GYTFTSYY (SEQ ID NO: 9); a heavy chain complementarity determining region 2 (CDR-H2) having the sequence INPSGGST (SEQ ID NO: 10); and a heavy chain complementarity determining region 3 (CDR-H3) having the sequence ARGSAYYYDFADY (SEQ ID NO: 11), and a light chain variable domain (VL_CD16a) comprising a light chain complementarity determining region 1 (CDR-L1) having the sequence NIGSKN (SEQ ID NO: 12), a light chain complementarity determining region 2 (CDR-L2) having the sequence QDN; and a light chain complementarity determining region 3 (CDR-L3) having the sequence QVWDNYSVL (SEQ ID NO: 13).

In some embodiments, the binding domain specifically binding CD16A of the bispecific antibody construct comprises a heavy chain variable domain (VH_CD16a) comprising a heavy chain complementarity determining region 1 (CDR-H1) having the sequence GYTFTSYY (SEQ ID NO: 17); a heavy chain complementarity determining region 2 (CDR-H2) having the sequence IEPMYGST (SEQ ID NO: 18); and a heavy chain complementarity determining region 3 (CDR-H3) having the sequence ARGSAYYYDFADY (SEQ ID NO: 19), and a light chain variable domain (VL_CD16a) comprising a light chain complementarity determining region 1 (CDR-L1) having the sequence NIGSKN (SEQ ID NO: 20), a light chain complementarity determining region 2 (CDR-L2) having the sequence QDN; and a light chain complementarity determining region 3 (CDR-L3) having the sequence QVWDNYSVL (SEQ ID NO: 21).

In some embodiments, the binding domain specifically binding CD16A of the bispecific antibody construct comprises a heavy chain variable domain (VH_CD16a) comprising a heavy chain complementarity determining region 1 (CDR-H1) having the sequence GYTFTNYY (SEQ ID NO: 25); a heavy chain complementarity determining region 2 (CDR-H2) having the sequence INPSGGVT (SEQ ID NO: 26); and a heavy chain complementarity determining region 3 (CDR-H3) having the sequence ARGSAYYYDFADY (SEQ ID NO: 27), and a light chain variable domain (VL_CD16a) comprising a light chain complementarity determining region 1 (CDR-L1) having the sequence NIGSKS (SEQ ID NO: 28), a light chain complementarity determining region 2 (CDR-L2) having the sequence QDK; and a light chain complementarity determining region 3 (CDR-L3) having the sequence QVWDDYIVL (SEQ ID NO: 29).

In some embodiments, the binding domain specifically binding CD16A of the bispecific antibody construct comprises a heavy chain variable domain (VH_CD16a) comprising a heavy chain complementarity determining region 1 (CDR-H1) having the sequence SYYMH (SEQ ID NO: 32); a heavy chain complementarity determining region 2 (CDR-H2) having the sequence AIEPRYGSTSYAQKFQG (SEQ ID NO: 33); and a heavy chain complementarity determining region 3 (CDR-H3) having the sequence GSAYYYDFADY (SEQ ID NO: 34), and a light chain variable domain (VL_CD16a) comprising a light chain complementarity determining region 1 (CDR-L1) having the sequence GGHNIGSKNVH (SEQ ID NO: 35), a light chain complementarity determining region 2 (CDR-L2) having the sequence QDNKRPS (SEQ ID NO: 36); and a light chain complementarity determining region 3 (CDR-L3) having the sequence QVWDNYNVL (SEQ ID NO: 37).

In some embodiments, the binding domain specifically binding CD16A of the bispecific antibody construct comprises a heavy chain variable domain (VH_CD16a) comprising a heavy chain complementarity determining region 1 (CDR-H1) having the sequence NYYMQ (SEQ ID NO: 38); a heavy chain complementarity determining region 2 (CDR-H2) having the sequence IINPSGGVTSYAQKFQG (SEQ ID NO: 39); and a heavy chain complementarity determining region 3 (CDR-H3) having the sequence GSAYYYDFADY (SEQ ID NO: 40), and a light chain variable domain (VL_CD16a) comprising a light chain complementarity determining region 1 (CDR-L1) having the sequence GGNNIGSKSVH (SEQ ID NO: 41), a light chain complementarity determining region 2 (CDR-L2) having the sequence QDKKRPS (SEQ ID NO: 42); and a light chain complementarity determining region 3 (CDR-L3) having the sequence QVWDDYIVL (SEQ ID NO: 43).

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 14; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 15.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 22; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 23.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 30; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 31.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 44; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 45.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 46; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 47.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 48; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 49.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 50; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 51.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 52; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 53.

In some embodiments, the binding domain specifically binding CD16A comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 54; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 55.

In some embodiments, the binding domain specifically binding CD16A comprises a heavy chain variable (VH) region comprising or consisting of SEQ ID NO: 14; and a light chain variable (VL) region comprising or consisting of SEQ ID NO: 15.

In some embodiments, the binding domain specifically binding CD16A comprises a heavy chain variable (VH) region comprising or consisting of SEQ ID NO: 22; and a light chain variable (VL) region comprising or consisting of SEQ ID NO: 23.

In some embodiments, the binding domain specifically binding CD16A comprises a heavy chain variable (VH) region comprising or consisting of SEQ ID NO: 30; and a light chain variable (VL) region comprising or consisting of SEQ ID NO: 31.

In some embodiments, the binding domain specifically binding CD16A comprises a heavy chain variable (VH) region comprising or consisting of SEQ ID NO: 44; and a light chain variable (VL) region comprising or consisting of SEQ ID NO: 45.

In some embodiments, the binding domain specifically binding CD16A comprises a heavy chain variable (VH) region comprising or consisting of SEQ ID NO: 46; and a light chain variable (VL) region comprising or consisting of SEQ ID NO: 47.

In some embodiments, the binding domain specifically binding CD16A comprises a heavy chain variable (VH) region comprising or consisting of SEQ ID NO: 48; and a light chain variable (VL) region comprising or consisting of SEQ ID NO: 49.

In some embodiments, the binding domain specifically binding CD16A comprises a heavy chain variable (VH) region comprising or consisting of SEQ ID NO: 50; and a light chain variable (VL) region comprising or consisting of SEQ ID NO: 51.

In some embodiments, the binding domain specifically binding CD16A comprises a heavy chain variable (VH) region comprising or consisting of SEQ ID NO: 52; and a light chain variable (VL) region comprising or consisting of SEQ ID NO: 53.

In some embodiments, the binding domain specifically binding CD16A comprises a heavy chain variable (VH) region comprising or consisting of SEQ ID NO: 54; and a light chain variable (VL) region comprising or consisting of SEQ ID NO: 55.

In some embodiments, the first binding domain is a variable domain (Fv). In some embodiments, the first binding domain is a single chain Fv (scFv). In some embodiments, the first binding domain is a Fab. In some embodiments, the first binding domain is a single chain diabody (scDb). In some embodiments, the first binding domain is a diabody (Db). In some embodiments, the first binding domain is a double Fab. Preferably, the first binding domain is a Fab.

In some embodiments, the binding domain specifically binding EGFR of the bispecific antibody construct comprises a heavy chain variable domain (VH_EGFR) comprising a heavy chain complementarity determining region 1 (CDR-H1) having the sequence GGSVSSGSYY (SEQ ID NO: 56); a heavy chain complementarity determining region 2 (CDR-H2) having the sequence IYYSGST (SEQ ID NO: 57); and a heavy chain complementarity determining region 3 (CDR-H3) having the sequence ARNPISIPAFDI (SEQ ID NO: 58), and a light chain variable domain (VL_ EGFR) comprising a light chain complementarity determining region 1 (CDR-L1) having the sequence NIGSKS (SEQ ID NO: 59), a light chain complementarity determining region 2 (CDR-L2) having the sequence YDS; and a light chain complementarity determining region 3 (CDR-L3) having the sequence QVWDTSSDHVL (SEQ ID NO: 60).

In some embodiments, the binding domain specifically binding EGFR comprises a heavy chain variable (VH) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 61; and a light chain variable (VL) region comprising or consisting of an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity to SEQ ID NO: 62.

In some embodiments, the binding domain specifically binding EGFR comprises a heavy chain variable (VH) region comprising or consisting of SEQ ID NO: 61; and a light chain variable (VL) region comprising or consisting of SEQ ID NO: 62.

In some embodiments, the second binding domain is a variable domain (Fv). In some embodiments, the second binding domain is a single chain Fv (scFv). In some embodiments, the second binding domain is a Fab. In some embodiments, the second binding domain is a single chain diabody (scDb). In some embodiments, the second binding domain is a diabody (Db). In some embodiments, the second binding domain is a double Fab. Preferably, the second binding domain is a scFv.

In some embodiments, the bispecific antibody construct binds to a target cell and an immune effector cell simultaneously, as described elsewhere herein.

In some embodiments, the bispecific antibody construct comprises a third domain comprising a half-life extension domain. In some embodiments, the half-life extension domain comprises a CH2 domain, wherein the Fcγ receptor binding domain is optionally silenced. In some embodiments, the half-life extension domain comprises a CH3 domain.

As used herein the term "half-life extensions domain" relates to a moiety that prolongs serum half-life of the antibody construct. The half-life extension domain may comprise a portion of an antibody, such as an Fc part of an immunoglobulin, a hinge domain, a CH2 domain, a CH3 domain, and/or a CH4 domain. Although less preferred, a half-life extension domain can also comprise elements that are not comprised in an antibody, such as an albumin binding peptide, an albumin binding protein, or transferrin to name only a few. A half-life extension domain preferably does not have an immune-modulatory function. If a half-life extension domain comprises a hinge, CH2 and/or CH3 domain, the half-life extension domain preferably does not essentially bind to an Fc receptor. This can e.g. be achieved through "silencing" of the Fcy receptor binding domain.

As used herein, "silencing" of the Fc or Fcy receptor binding domain refers to any modification that reduces binding of a CH2 domain to an Fc receptor, in particular an Fcy receptor. Such modification can be done by replacement and/or deletion of one or more amino acids that are involved in Fc(γ) receptor-binding. Such mutations are well known in the art and have e.g. been described by Saunders (2019, Front. Immunol. 10:1296). For example, a mutation can be located at any one of the positions 233, 234, 235, 236, 237, 239, 263, 265, 267, 273, 297, 329, and 331. Examples for such mutations are: deletion of Glu 233 -> Pro, Glu 233, Leu 234 -> Phe, Leu 234 -> Ala, Leu 234 -> Gly, Leu 234 -> Glu, Leu 234 -> Val, deletion of Leu 234, Leu 235 -> Glu, Leu 235 -> Ala, Leu 235 -> Arg, Leu 235 -> Phe, deletion of Leu 235, deletion of Gly 236, Gly 237 -> Ala, Ser 239 -> Lys, Val 263 -> Leu, Asp 265 -> Ala, Ser 267 -> Lys, Val 273 -> Glu, Asn 297 -> Gly, Asn 297 -> Ala, Lys 332 -> Ala, Pro 329 -> Gly, Pro 331 -> Ser and combinations thereof. Preferably, such a modification comprises one or both of Leu 234 -> Ala and Leu 235 -> Ala (also known as "LALA" mutation). Preferably, such a modification further comprises a Pro 329 -> Gly mutation, also known as "LALA-PG" mutation (Leu 234 -> Ala, Leu 235 -> Ala, and Pro 329 -> Gly). Preferably, such a modification comprises 1, 2, or 3 of the mutations Leu 234 -> Phe, Leu 235 -> Glu, and Asp 265 -> Ala, more preferably all three of these mutations. The combination Leu 234 -> Phe, Leu 235 -> Glu, and Asp 265 -> Ala, which is a preferred modification in the context of the present invention, is also known as "FEA" mutation. Preferably, such a modification further comprises Asn 297 - > Gly. Such a preferred modification comprises the mutations Leu 234 -> Phe, Leu 235 -> Glu, Asp 265 -> Ala, and Asn 297 -> Gly.

In some embodiments, the half-life extension domain may comprise two CH3 domains. The half-life extension domain may comprise a hinge domain. The half-life extension domain may comprise two hinge domains. The half-life extension domain may comprise a CH2 domain and a CH3 domain. In such a case, the CH2 domain and CH3 domain are preferably fused to each other, preferably in the (amino to carboxyl) order CH2 domain - CH3 domain. The half-life extension domain may comprise a hinge domain and a CH2 domain. In such a case, the hinge domain and the CH2 domain are preferably fused to each other, preferably in the (amino to carboxyl) order hinge domain - CH2 domain. The half-life extension domain may comprise a hinge domain, a CH2 domain, and a CH3 domain. In such a case, the hinge domain, the CH2 domain, and CH3 domain are preferably fused to each other, preferably in the (amino to carboxyl) order hinge domain - CH2 domain - CH3 domain. The half-life extending domain may comprise two hinge domain - CH2 domain elements, two CH2 domain - CH3 domain elements, or two hinge domain - CH2 domain - CH3 domain elements. In such a case the two fusions may be located on two different polypeptide strands. Alternatively, the fusions can be located on the same polypeptide strand. An illustrative example for two hinge domain - CH2 domain - CH3 domain elements that are located on the same polypeptide strand is the "single chain Fc" or "scFc" format. Here, both hinge-CH2-CH3 subunits are fused together via a linker that allows assembly of a Fc domain. A preferred linker for this purpose is a glycine serine linker, which preferably comprises from about 20 to about 40 amino acids. Preferred glycine serine linkers may have one or more repeats of GGS, GGGS (SEQ ID NO: 72), or GGGGS (SEQ ID NO: 5). Such linker preferably comprises 4-8 repeats (e.g. 4, 5, 6, 7, or 8 repeats) of GGGGS. Such a linker is preferably (GGGGS)₆, (SEQ ID NO 8). Further scFc constant domains are known in the art and *inter alia* described in WO 2017/134140.

Generally, the bispecific antibody constructs used in accordance with the present invention can be monovalent, bivalent, trivalent, or have an even higher valency for any one of the first target (CD16A) and the second target (EGFR). The antibody constructs of the disclosure may thus comprise one, two, three, or even more of any one of the first binding domain and the second binding domain. It is preferred for the antibody construct of the invention that it is at least monovalent for the first target (CD16A) and at least monovalent for the second target (EGFR). It is also preferred for the antibody construct of the invention that it is at least monovalent for the first target (CD16A) and bivalent for the second target (EGFR). It is further preferred for the antibody construct of the invention that it is at least bivalent for the first target (CD16A) and at least bivalent for the second target (EGFR). It also preferred for the antibody construct of the invention that it is at least bivalent for the first target (CD16A) and at least trivalent for the second target (EGFR). It also preferred for the antibody construct of the invention that it is at least bivalent for the first target (CD16A) and at least monovalent for the second target (EGFR). Most preferred, the antibody construct of the invention is bivalent for the first target (CD16A) and bivalent for the second target (EGFR).

In some embodiments, the bispecific antibody construct in accordance with the present invention comprises at least one first binding domain and at least one second binding domain. In some embodiments, the bispecific antibody construct comprises at least one first binding domains and at least two second binding domains. It is further preferred for the bispecific antibody construct that it comprises at least two first binding domains and at least two second binding domains. It is further preferred for the bispecific antibody construct that it comprises at least two first binding domains and at least three second binding domains. It is further preferred for the bispecific antibody construct that it comprises at least two first binding domains and at least one second binding domain. Most preferred, the bispecific antibody construct comprises two first binding domains and two second binding domains.

In some embodiments, the first binding domain or the second binding domain of the bispecific antibody construct may be fused to a constant domain of an antibody via a linker. Such a linker is preferably a short linker, which preferably has a length of about 10 nm or less, preferably about 9 nm or less, preferably about 8 nm or less, preferably about 7 nm or less, preferably about 6 nm or less, preferably about 5 nm or less, preferably about 4 nm or less, or even less. The length of the linker is preferably determined as described by Rossmalen et al Biochemistry 2017, 56, 6565-6574, which also describes suitable linkers that are well known to the skilled person. An example for a suitable linker is a glycine serine linker or a serine linker, which preferably comprise no more than about 75 amino acids, preferably not more than about 50 amino acids. In illustrative example, a suitable linker comprises one or more (e.g. 1, 2, 3, 4, 5, 6, 7, or 8) GGGGS sequences (SEQ ID NO: 5), such as (GGGGS)₄ (SEQ ID NO: 7), (GGGGS)₆ (SEQ ID NO: 8), or preferably (GGGGS)₂ (SEQ ID NO: 6). Other illustrative examples for linkers are shown in SEQ ID NOs: 1-8 and 72.

In some embodiments, the bispecific antibody construct may also comprise additional domains, which are e.g. helpful in the isolation of the molecule or relate to an adapted pharmacokinetic profile of the molecule. Domains helpful for the isolation of an antibody construct may be selected from peptide motives or secondarily introduced moieties, which can be captured in an isolation method, e.g. an isolation column. Non-limiting embodiments of such additional domains comprise peptide motives known as Myc-tag, HAT-tag, HA-tag, TAP-tag, GST-tag, chitin binding domain (CBD-tag), maltose binding protein (MBP-tag), Flag-tag, Strep-tag and variants thereof (e.g. StrepII-tag) and His-tag. All herein disclosed antibody constructs characterized by the identified CDRs may comprise a His-tag domain, which is generally known as a repeat of consecutive His residues in the amino acid sequence of a molecule, preferably of five, and more preferably of six His residues (hexa-histidine). The His-tag may be located e.g. at the N- or C-terminus of the antibody construct, preferably it is located at the C-terminus. Most preferably, a hexa-histidine tag is linked via peptide bond to the C-terminus of the antibody construct according to the invention. Additionally, a conjugate system of PLGA-PEG-PLGA may be combined with a poly-histidine tag for sustained release application and improved pharmacokinetic profile. In some embodiments the histidine tag is a hexa-histidine tag (SEQ ID NO: 73).

Amino acid sequence modifications of the antibody constructs described herein are also contemplated, as long as the minimal structural limitations of the first binding domain of the antibody construct of the present invention are maintained. For example, it may be desirable to improve the binding affinity and/or other biological properties of the antibody construct. Amino acid sequence variants of the antibody constructs are prepared by introducing appropriate nucleotide changes into the antibody constructs nucleic acid, or by peptide synthesis. All of the below described amino acid sequence modifications should result in an antibody construct which still retains the desired biological activity (i.e. at least binding to CD16A and EGFR) of the unmodified parental molecule.

Amino acid modifications include, for example, deletions from, and/or insertions into, and/or substitutions of, residues within the amino acid sequences of the antibody constructs. Any combination of deletion, insertion, and substitution is made to arrive at the final construct, provided that the final construct possesses the desired characteristics. The amino acid changes also may alter post-translational processes of the antibody constructs, such as changing the number or position of glycosylation sites.

Generally, if amino acids are substituted in one or more or all of the CDRs of the heavy and/or light chain, it is preferred that the then-obtained "substituted" sequence is at least 60% or at least 65%, more preferably at least 70% or at least 75%, even more preferably at least 80% or at least 85%, and particularly preferably at least 90% or at least 95% identical to the "original" CDR sequence. This means that it is dependent of the length of the CDR to which degree it is identical to the "substituted" sequence. For example, a CDR having 5 amino acids is preferably at least 80% identical to its substituted sequence in order to have at least one amino acid substituted. Accordingly, the CDRs of the antibody construct may have different degrees of identity to their substituted sequences, e.g., CDRL1 may have at least 80%, while CDRL3 may have at least 90%. Preferred substitutions (or replacements) are conservative substitutions. However, any substitution (including non-conservative substitution) is envisaged as long as the antibody construct retains its capability to bind to CD16A via the first binding domain, and/or to the target cell surface antigen via the second binding domain and/or the CDRs of the second binding domain have an identity to the then substituted sequence (at least 60% or at least 65%, more preferably at least 70% or at least 75%, even more preferably at least 80% or at least 85%, and particularly preferably at least 90% or at least 95% identical to the "original" CDR sequence). Conservative substitutions are shown in **Table 1** under the heading of "preferred substitutions". If such substitutions result in a change in biological activity, then more substantial changes, denominated "exemplary substitutions" in Table 1, or as further described below in reference to amino acid classes, may be introduced and the products screened for a desired characteristic.

**Table 1: Amino acid substitutions**

| **Original** | **Exemplary Substitutions** | **Preferred Substitutions** |
|---|---|---|
| Ala (A) | val, leu, ile | va! |
| Arg (R) | Iys, gln, asn | lys |
| Asn (N) | gln, his, asp, Iys, arg | gln |
| Asp (D) | glu, asn | glu |
| Cys (C) | ser, ala | ser |
| Gln (Q) | asn, glu | asn |
| Glu (E) | asp, gln | asp |
| Gly (G) | ala | ala |
| His (H) | asn, gln, Iys, arg | a rg |
| Ile(I) | leu, val, met, ala, phe | leu |
| Leu (L) | norleucine, i!e, va!, met, ala | lie |
| Lys (K) | arg, gln, asn | a rg |
| Met (M) | leu, phe, i!e | leu |
| Phe (F) | leu, val, i!e, ala, tyr | tyr |
| Pro (P) | ala | ala |
| ser (S) | thr | thr |
| Thr (T) | ser | ser |
| Trp (W) | tyr, phe | tyr |
| Tyr (Y) | trp, phe, thr, ser | phe |
| Val (V) | ile, leu, met, phe, ala | leu |

Non-conservative substitutions will entail exchanging a member of one of these classes for another class. Any cysteine residue not involved in maintaining the proper conformation of the antibody construct may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

In preferred embodiments, the second binding domain of the bispecific antibody construct is preferably a scFv fragment that is fused to a C terminus of a Fc domain, preferably via the VH domain of the scFv. Accordingly, the arrangement of the polypeptide chain (from N to C) is preferably ...-CH2-CH3-VH-VL, optionally with a linker between the Fc and the scFv. The first binding domain can be located at any suitable position of the antibody construct. Where the antibody construct comprises a Fc region, the first binding domain can be located N terminal of the Fc region, either directly or linked via at least a part of a hinge domain. Other linkers disclosed herein can also be used to link the first binding domain to the Fc domain. A hinge domain is however preferred for this purpose. The first binding domain can be any suitable structure disclosed herein, while a Fab structure is preferred. The first binding domain and the third domain can be fused to each other in a way, that they form a conventional immunoglobuline. Such immunoglobulin may have two heavy chains and two light chains of an immunoglobulin as described elsewhere herein.

The bispecific antibody construct used in accordance with the present invention is preferably in a format referred to as "scFv-IgAb" herein. Such an antibody construct comprises an immunoglobulin that has one scFv fragment fused to the C terminus of each of the two heavy chains, optionally via a linker, preferably a linker disclosed herein. Said scFvs form the second binding domain (EGFR). Two first binding domains (CD16A) are formed by the binding sites of the immunoglobulin. The scFv-IgAb format may comprise four polypeptide chains, two light chains in the arrangement VL(CD16A)-CL, and two heavy chains each fused to a scFv in the arrangement VH(CD16A)-CH1-hinge-CH2-CH3-VH(EGFR)-VL(EGFR) (or less preferred VH(CD16A)-CH1-hinge-CH2-CH3-VL(EGFR)-VH(EGFR)). Illustrative examples for such antibody constructs are shown in SEQ ID NOs: 63 and 64 or 65 and 66.

The bispecific antibody construct used in accordance with the present invention may comprise an immunoglobulin that has one scFv fragment fused to the C terminus of each of the two heavy chains and to the C terminus of each of the two light chains, optionally via a linker, preferably a linker disclosed herein. Said scFvs form the second binding domain (EGFR). Two first binding domains (CD16A) are formed by the binding sites of the immunoglobulin. The scFv-IgAb format may comprise four polypeptide chains, two light chains in the arrangement VL(CD16A)-CL-VH(EGFR)-VL(EGFR), and two heavy chains each fused to a scFv in the arrangement VH(CD16A)-CH1-hinge-CH2-CH3-VH(EGFR)-VL(EGFR) (or less preferred VH(CD16A)-CH1-hinge-CH2-CH3-VL(EGFR)-VH(EGFR)). Illustrative examples for such antibody constructs are shown in SEQ ID NOs: 63 and 68 or 65 and 68.

The bispecific antibody construct used in accordance with the present invention may be in the form of a single chain diabody. As such, the bispecific antibody construct may comprise one single polypeptide chain, in the arrangement VH(EGFR)-VL(CD16A)-VH(CD16A)-VL(EGFR). An illustrative example for such an antibody construct is shown in SEQ ID NO: 67.

In preferred embodiments, the bispecific antibody construct used in accordance with the present invention is a bispecific antibody construct comprising (a) a first binding domain which is capable of specifically binding CD16A, comprising: a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13); and (b) a second binding domain which is capable of specifically binding EGFR, comprising a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).

In preferred embodiments, the bispecific antibody construct used in accordance with the present invention is a bispecific antibody construct comprising (a) a first binding domain comprising a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13), wherein said first binding domain is a Fab; (b) a second binding domain comprising a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60), wherein said second binding domain is a scFv; and (c) a third domain comprising two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.

In preferred embodiments, the bispecific antibody construct used in accordance with the present invention is a bispecific antibody construct comprising (a) a first binding domain which is capable of specifically binding CD16A, comprising: a VH region as depicted in SEQ ID NO: 14 and a VL region as depicted in SEQ ID NO: 15; and (b) a second binding domain which is capable of specifically binding EGFR, comprising a VH region as depicted in SEQ ID NO: 61 and a VL region as depicted in SEQ ID NO: 62.

In preferred embodiments, the bispecific antibody construct used in accordance with the present invention is a bispecific antibody construct comprising (a) a first binding domain comprising a VH region as depicted in SEQ ID NO: 14 and a VL region as depicted in SEQ ID NO: 15, wherein said first binding domain is a Fab; (b) a second binding domain comprising a VH region as depicted in SEQ ID NO: 61 and a VL region as depicted in SEQ ID NO: 62, wherein said second binding domain is a scFv; and (c) a third domain comprising two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.

In preferred embodiments, the bispecific antibody construct used in accordance with the present invention is an antibody construct comprising or consisting of amino acid sequences having at least 80%, at least 85%, at least 90%, at least 95%, at least 98%, or at least 99% sequence identity to the amino acid sequences set forth in SEQ ID NOs: 63 and 64; SEQ ID NOs: 65 and 66; SEQ ID NO: 67; SEQ ID NOs: 63 and 68; or SEQ ID NOs: 65 and 68. Preferably the bispecific antibody construct used in accordance with the present invention is an antibody construct comprising or consisting of amino acid sequences having at least 80%, at least 85%, at least 90%, at least 95%, at least 98%, or at least 99% sequence identity to the amino acid sequences set forth in SEQ ID NOs: 63 and 64.

In preferred embodiments, the bispecific antibody construct used in accordance with the present invention is an antibody construct comprising or consisting of amino acid sequences set forth in SEQ ID NOs: 63 and 64; SEQ ID NOs: 65 and 66; SEQ ID NO: 67; SEQ ID NOs: 63 and 68; or SEQ ID NOs: 65 and 68. Preferably the bispecific antibody construct used in accordance with the present invention is an antibody construct comprising or consisting of amino acid sequences set forth in SEQ ID NOs: 63 and 64. In this respect is envisaged that the bispecific antibody construct is preferably AFM24.

As mentioned before, the bispecific antibody construct and/or the VEGFR-2 inhibitor are used in the method of treating a tumor in a subject according to the present invention, wherein the method comprises administering to the subject -in addition to administering the bispecific antibody construct and theVEGFR-2 inhibitor - also an immune checkpoint inhibitor (ICI).

Immune checkpoint inhibitors (ICIs) can be used to treat many types of cancers, including non-small cell lung cancer, melanoma, renal cell carcinoma, head and neck cancer, and gastrointestinal cancer. ICIs function to block the inactivity of immune cells towards tumor cells. Normally, immune cells are controlled under homeostatic conditions using immune checkpoints which maintain a balance between pro-inflammatory and anti-inflammatory signals. ICIs can promote anti-cancer immune responses, shifting the balance to proinflammatory, by binding to immune inhibitory receptors, such as CTLA-4, PD-1, and PD-L receptors on the surface of T-lymphocytes, and allowing activation of the T-lymphocytes towards the tumor. By blocking the immune inhibitory receptors, the ICIs overcome tumor-mediated immune inhibition, and facilitate a local inflammatory anti-tumor effect.

Generally, an immune checkpoint inhibitor that can be used in accordance with the present invention can be one that binds to CTLA-4, PD-1, or PD-L receptors. In some examples, such an immune checkpoint inhibitor binds to a receptor on the surface of T-lymphocytes, and allowing activation of the T-lymphocytes towards the tumor. Preferably, an immune checkpoint inhibitor that can be used in accordance to the present disclosure is one that targets PD-1/PD-L1, which are immune-checkpoint molecules that inhibit the activation of antigen-presenting cells and T cells (priming phase) and the cytotoxic functions of T cells (effector phase). Preferred immune checkpoint inhibitors are PD-L1 inhibitors and PD-1 inhibitors, with PD-L1 inhibitors being more preferred. The immune checkpoint inhibitor is preferably an antibody or an antibody construct. Hence, preferred immune checkpoint inhibitors include anti-PD-1 antibodies and anti-PD-L1 antibodies. Most preferred are anti-PD-L1 antibodies.

It is envisioned by the present disclosure, that the immune checkpoint inhibitor may be an antibody that comprises means (e.g., an antigen binding site) for specifically binding PD-1 or PD-L1, preferably PD-L1.

Several anti-PD-1 antibodies and anti-PD-L1 antibodies are known to the skilled person and their use according to the present disclosure is contemplated. Examples of such antibodies are given in the following. Nivolumab (OPDIVO^{™}, Bristol Myers Squibb) is a human IgG4 monoclonal antibody that blocks PD-1. See WO2006121168A1 (Ono Pharmaceutical Co. LTD.). Atezolizumab (Tecentriq^{™}, Genetech) is a humanized, monoclonal antibody of IgG1 isotype against PD-L1 and is used for the treatment of various cancer types. See WO2010077634A1 (Genetech). Pembrolizumab (Keytruda^{™}, Merck), is a humanized IgG4 isotype anti antibody against the PD-1 receptor. See WO2008156712 A1 (N. V. Organon). Cemiplimab (Libtayo^{™}, Regeneron Pharmaceuticals, Inc.) is a monoclonal antibody that binds to PD-1. See WO2015112800A1, H4H7798N (Regeneron Pharmaceuticals, Inc). Dostarlimab (TSR-042, JEMPERLI^{™}, Glaxo Smith Kline) is a humanized, monoclonal antibody of the IgG 4κ isotype that binds to PD-1. Durvalumab (Imfinzi^{™}, Medimmune/AstraZeneca) is a human immunoglobulin G1 kappa (IgG1κ) monoclonal antibody that blocks the interaction of PD-L1 with PD-1.

Other anti-PD-1 antibodies include pidilizumab, MED10608, BI 754091, PF-0680159, spartalizumab, camrelizumab, JNJ-63723283, and MCLA-134.

Other anti-PD-L1 antibodies include H2M8314N, avelumab, MDX-1105, LY3300054, FAZ053, STI-1014, CX-072, KN035, and CK-301.

An immune checkpoint inhibitor that can be used in accordance with the present invention, may be selected from the group consisting of nivolumab, atezolizumab, pembrolizumab, cemiplimab, H4H7798N, dostarlimab, durvalumab, pidilizumab, MED10608, BI 754091, PF-0680159, spartalizumab, camrelizumab, JNJ-63723283, MCLA-134, H2M8314N, avelumab, MDX-1105, LY3300054, FAZ053, STI-1014, CX-072, KN035, and CK-301. Optionally, the immune checkpoint inhibitor is selected from the group consisting of nivolumab atezolizumab, pembrolizumab cemiplimab, H4H7798N, dostarlimab, and durvalumab. Preferably, the immune checkpoint inhibitor is nivolumab, atezolizumab or pembrolizumab. More preferably, the immune checkpoint inhibitor is atezolizumab. Atezolizumab is a known, published humanized monoclonal antibody used to prevent the interaction of PD-L1 and PD-1, see e.g. DrugBank Accession Number DB11595.

In accordance with some embodiments, the immune checkpoint inhibitor may be an antibody comprising means for for specifically binding PD-L1.

Turning now to the VEGFR-2-inhibitor, in accordance with some embodiments, the VEGFR-2 inhibitor may be an anti-VEGFR-2 antibody.

It is envisaged that the VEGFR-2 inhibitor may be selected from the group consisting of ramucirumab, bevazirumab, and aflibercept.

Preferably, the VEGFR-2 inhibitor may be ramucirumab. Ramucirumab is a known, published human monoclonal antibody (IgG1) against vascular endothelial growth factor receptor 2 (VEGFR2), see e.g. DrugBank Accession Number DB05578.

The term "pharmaceutical composition" relates to a composition which is suitable for administration to a patient, preferably a human patient. In some embodiments, the pharmaceutical composition administered to said patient comprises one or a plurality of the bispecific antibody construct(s) described. Accordingly, in the context of the present invention, the bispecific antibody construct(s) described herein may be comprised in a first pharmaceutical composition and the VEGFR-2 inhibitor described herein may be comprised in a second pharmaceutical composition, and the immune checkpoint inhibitor described herein may be comprised in a third pharmaceutical composition which may all be administered to said patient in the course of the therapeutic treatment. In some embodiments, the VEGFR-2 inhibitor and the bispecific antibody construct(s) may be comprised in one pharmaceutical composition that is administered to said subject in the course of the therapeutic treatment. In some embodiments, the immune checkpoint inhibitor and the bispecific antibody construct(s) may be comprised in one pharmaceutical composition that is administered to said subject in the course of the therapeutic treatment. In some embodiments, the immune checkpoint inhibitor and the VEGFR-2 inhibitor may be comprised in one pharmaceutical composition that is administered to said subject in the course of the therapeutic treatment. In some embodiments, the VEGFR-2 inhibitor, the bispecific antibody construct(s), and the immune checkpoint inhibitor may be comprised in one pharmaceutical composition that is administered to said subject in the course of the therapeutic treatment

Preferably, the pharmaceutical composition(s) used in the context of the present invention comprises suitable formulations of one or more (pharmaceutically effective) carriers, stabilizers, excipients, diluents, solubilizers, surfactants, emulsifiers, preservatives and/or adjuvants. Acceptable constituents of the composition are preferably nontoxic to recipients at the dosages and concentrations employed. Pharmaceutical compositions that can be used in the context of the present invention include, but are not limited to, liquid, frozen, and lyophilized compositions. As used herein the language "pharmaceutically acceptable carrier" includes saline, solvents, dispersion media, coatings, antibacterial and antifungal agents, isotonic and absorption delaying agents, and the like, compatible with pharmaceutical administration. The use of such media and agents in pharmaceutical compositions is well known in the art, and the compositions comprising such carriers can be formulated by well-known conventional methods.

Pharmaceutical compositions are typically formulated to be compatible with its intended route of administration. Examples of routes of administration include parenteral, e.g., intravenous, intradermal, subcutaneous, oral, transdermal (topical), transmucosal, and rectal administration. In the context of the means and methods of the present invention it is particularly envisaged that the pharmaceutical composition(s) comprising the VEGFR-2 inhibitor, and/or the bispecific antibody construct, and/or the immune checkpoint inhibitor is suitable for intravenous administration.

Methods of formulating suitable pharmaceutical compositions are known in the art, see, e.g., Remington: The Science and Practice of Pharmacy, 21st ed., 2005; and the books in the series Drugs and the Pharmaceutical Sciences: a Series of Textbooks and Monographs (Dekker, NY). For example, solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent that delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying, which yield a powder of the active ingredient plus any additional desired ingredient from a previously sterile-filtered solution thereof.

As set forth above, in the context of the present invention, the bispecific antibody described herein, e.g. a pharmaceutical compositions comprising the bispecific antibody described herein, is administered to the patient in combination with a VEGFR-2 inhibitor as described herein, e.g., a pharmaceutical composition comprising said VEGFR-2 inhibitor as described herein, and in further combination with an immune checkpoint inhibitor as described herein, e.g., a pharmaceutical composition comprising said immune checkpoint inhibitor as described herein. In some embodiments, the bispecific antibody is administered together with the VEGFR-2 inhibitor and/or together with the immune checkpoint inhibitor as part of one pharmaceutical composition. In some embodiments, the bispecific antibody may be administered separately from the VEGFR-2 inhibitor and/or separately from the immune checkpoint inhibitor, e.g., as part of a separate pharmaceutical composition. According to this embodiment, also the VEGFR-2 inhibitor may be administered separately from the immune checkpoint inhibitor e.g., as part of a separate pharmaceutical composition. The bispecific antibody constructs comprising at least a CD16A and a EGFR binding domain as described herein can be administered prior to, subsequent to, or simultaneously with administration of the VEGFR-2 inhibitor and/or the immune checkpoint inhibitor. In some embodiments, the VEGFR-2 inhibitor can be administered prior to, subsequent to, or simultaneously with administration of the bispecific antibody construct and/or the immune checkpoint inhibitor. In some embodiments, the immune checkpoint inhibitor may be administered prior to, subsequent to, or simultaneously with administration of the VEGFR-2 inhibitor and/or the bispecific antibody construct.

In some embodiments, the bispecific antibody construct may be administered to the subject once every about 6 to about 8 days, or once every about 7 days.

In some embodiments, the bispecific antibody construct is administered at a dose in the range of about 300 to about 800 mg, about 400 to about 550 mg, about 420 to about 530 mg, about 450 to about 500 mg, or about 480 mg.

According to some embodiments, the VEGFR-2 inhibitor is administered to the subject once every about 1 to about 3 weeks, once every about 2 weeks, once every about 12 to about 16 days, once every about 13 to about 15 days, or once every about 14 days.

In some embodiments, the VEGFR-2 inhibitor is administered at a dose in the range of about 5 to about 15 mg, about 8 to about 12 mg, about 9 to about 11 mg, or about 10 mg.

In some embodiments, the immune checkpoint inhibitor is administered to the subject once every about 1 to about 3 weeks, once every about 2 weeks, once every about 12 to about 16 days, once every about 13 to about 15 days, or once every about 14 days.

According to some embodiments, the immune checkpoint inhibitor is administered at a dose in the range of about 500 to about 1200 mg, about 700 to about 1000 mg, about 800 to about 900 mg, or about 840 mg.

In some embodiments, the bispecific antibody construct as described herein, the immune checkpoint inhibitor as described herein and/or the VEGFR-2 inhibitor as described herein are administered to the patient as part of a treatment cycle that spans multiple days. In some embodiments, the bispecific antibody construct, the VEGFR-2 inhibitor and/or the immune checkpoint inhibitor are administered to the patient as part of a treatment regimen that comprises one or more treatment cycles.

In some embodiments, the treatment regimen continues until the patient's disorder (i.e. the tumor disease) progresses, or until the doses are discontinued due to the patient's intolerance of the bispecific antibody construct, of the VEGFR-2 inhibitor, of the immune checkpoint inhibitor, or due to the patient's intolerance of at least two of them, or due to the patient's intolerance of all three compounds.

The ability to offer repeat treatment/dosing may allow patients to experience or maintain a deeper or prolonged response from the therapy. Thus, in some embodiments, the patient receives multiple doses, e.g., two or more doses or at least one subsequent dose, of the bispecific antibody construct, of the VEGFR-2 inhibitor, and/or of the immune checkpoint inhibitor. In some embodiments, two, three, four, five, six, seven, eight, nine, or ten doses are administered to a subject. In some embodiments, the at least one subsequent dose comprises a second dose. In some embodiments, the at least one subsequent dose comprises a second dose and a third dose. In some embodiments, the at least one subsequent dose comprises a second, third, and fourth dose. In some embodiments, the at least one subsequent dose comprises a second, third, fourth, and fifth dose. In some embodiments, the at least one subsequent dose comprises a second, third, fourth, fifth, and sixth dose. In some embodiments, the at least one subsequent dose comprises a second, third, fourth, fifth, sixth, and seventh dose. In some embodiments, the at least one subsequent dose comprises a second, third, fourth, fifth, sixth, seventh, and eighth dose. In some embodiments, a patient can receive response-based dosing, during which the patient continues to receive the bispecific antibody construct, of the VEGFR-2 inhibitor, and/or of the immune checkpoint inhibitor for as long as the patient derives a benefit. The number of doses and the number of the bispecific antibody construct, of the VEGFR-2 inhibitor, and/or of the immune checkpoint inhibitor administered in each dose can also be tailored to the individual patient. In some embodiments, the number of bispecific antibody construct, of the VEGFR-2 inhibitor, and or of the immune checkpoint inhibitor administered to the subject in the additional or subsequent dose or doses are the same as or similar to the first dose. Thus, the therapies described herein can be tailored to each patient based on that patient's own response. In some cases, the therapy can be terminated if the patient no longer derives a benefit from therapy. In some cases, the therapy can also be reinitiated if the patient relapses.

In some embodiments, the dosing schedule can vary over the course of the therapy. Thus, for example, the patient can receive a first series (or cycle) of doses every 1, 2, 3, 4, 5, 6, 7, 8, 9, 10, 11, 12, 13, 14, 15, or 16 weeks and a second series of doses every 1, 2, 3, 4, 5, 6, 7, 8, 9, 10, 11, 12, 13, 14, 15, or 16 weeks, wherein the time between the first series of doses and the second series of doses is different. In some embodiments, the patient receives 1, 2, 3, 4, 5, 6, 7, 8, 9, or 10 doses in the first series of doses and 1, 2, 3, 4, 5, 6, 7, 8, 9, or 10 doses in the second series of doses, wherein the number of doses in the first series and the second series of doses can be the same or different. Thus, for example, a patient may receive four doses administered every other week in the first series of doses and four doses administered every 12 weeks in the second series of doses.

In some embodiments, the treatment regimen comprises a treatment break (e.g., a period without administration of the bispecific antibody construct, of the VEGFR-2 inhibitor, and/or of the immune checkpoint inhibitor) between treatment cycles. In some embodiments, the treatment break is from 1 to 8 weeks, e.g., from 1 to 8, 1 to 7, 1 to 6, 1 to 5, 1 to 4, 1 to 3, 1 to 2, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, or 7 to 8 weeks. In some embodiments, the treatment break is at least 1, 2, 3, 4, 5, 6, 7, or 8 weeks. In some embodiments, the treatment break is or is about 1, 2, 3, 4, 5, 6, 7, or 8 weeks.

In some embodiments, the treatment cycle is from 2 to 60 days, e.g., from 2 to 50, 2 to 40, 2 to 30, 2 to 20, 2 to 10, 2 to 5, 5 to 60, 5 to 50, 5 to 40, 5 to 30, 5 to 20, 5 to 10, 10 to 60, 10 to 50, 10 to 40, 10 to 30, 10 to 20, 20 to 60, 20 to 50, 20 to 40, 20 to 30, 30 to 60, 30 to 50, 30 to 40, 40 to 60, 40 to 50, or 50 to 60 days. In some embodiments, the treatment cycle is from 1 to 8 weeks (e.g., 1 to 7, 1 to 6, 1 to 5, 1 to 4, 1 to 3, 1 to 2, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, 5 to 8, 5 to 7, 5 to 6, 6 to 8, 6 to 7, or 7 to 8 weeks). In some embodiments, the treatment cycle is or is about 1, 2, 3, 4, 5, 6, 7, or 8 weeks.

In some embodiments, the treatment regimen comprises from 1 to 5 treatment cycles, e.g., 1 to 4, 1 to 3, 1 to 2, 2 to 5, 2 to 4, 2 to 3, 3 to 5, 3 to 4, or 4 to 5 treatment cycles. In some embodiments, the treatment regimen comprises 1, 2, 3, 4, or 5 treatment cycles. In some embodiments, the treatment cycles of a treatment regimen are the same (e.g., follow the same dosing and timing schedules). In some embodiments, the treatment cycles of a treatment regimen are different (e.g., follow different dosing and timing schedules).

In some embodiments, the treatment cycle comprises multiple administrations of the bispecific antibody construct, of the VEGFR-2 inhibitor, and/or of the immune checkpoint inhibitor as described herein. As part of a treatment cycle, the doses of the bispecific antibody construct, of the VEGFR-2 inhibitor, and/or of the immune checkpoint inhibitor are, in some cases, administered together (e.g., simultaneously or in succession during a single treatment day). In some cases, the doses of the bispecific antibody construct, of the VEGFR-2 inhibitor, and/or of the immune checkpoint inhibitor are administered together as part of one pharmaceutical composition. In other cases, the doses of the bispecific antibody construct, of the VEGFR-2 inhibitor, and/or of the immune checkpoint inhibitor are administered separately (e.g., on different treatment days), i.e. as parts of different pharmaceutical compositions. In other cases, the doses of two of the group consisting of the bispecific antibody construct, the VEGFR-2 inhibitor, and the immune checkpoint inhibitor are administered together as part of one pharmaceutical composition, and the doses of the remaining compound selected from the group consisting of the bispecific antibody construct, the VEGFR-2 inhibitor, and the immune checkpoint inhibitor may be administered separately (e.g., on different treatment days), i.e. as parts of different pharmaceutical compositions.

According to some embodiments, a cycle of administering consists of administering the bispecific antibody construct molecule 4 times, administering the VEGFR-2 inhibitor 2 times, and administering the immune checkpoint inhibitor 2 times, and treatment of the subject is carried out for at least one cycle.

For example, a cycle of administering may be about 4 weeks.

For example, administering of the VEGFR-2 inhibitor and administering of the immune checkpoint inhibitor may be at the same time or about at the same time.

In accordance with some embodiments, a dose of the VEGFR-2 inhibitor is concurrently administered with a dose of the bispecific antibody construct.

According to some embodiments, a dose of the bispecific antibody molecule and/or a dose the VEGFR-2 inhibitor may be administered as split-day dosing on two consecutive days.

According to some embodiments, the first dose of the bispecific antibody molecule and the first dose of VEGFR-2 inhibitor during the first cycle are administered as split-day dosing on two consecutive days.

According to some embodiments, the immune checkpoint inhibitor is administered in the first and third week of each cycle.

According to some embodiments, a dose the immune checkpoint inhibitor and a dose of the bispecific antibody construct are administered to the subject at the same time, or about the same time.

According to some embodiments, a dose the immune checkpoint inhibitor is concurrently administered with a dose of the bispecific antibody construct.

According to some embodiments, a dose of the bispecific antibody molecule and/or a dose the immune checkpoint inhibitor may be administered as split-day dosing on two consecutive days.

In some embodiments, the first dose of the bispecific antibody molecule and the first dose of immune checkpoint inhibitor during the first cycle are administered as split-day dosing on two consecutive days.

In some embodiments, the VEGFR-2 inhibitor and the immune checkpoint inhibitor are administered in the first and third week of each cycle.

For example, a dose the VEGFR-2 inhibitor, a dose of the immune checkpoint inhibitor and a dose of the bispecific antibody construct may be administered to the subject at the same time, or about the same time.

It is further envisaged that a dose the VEGFR-2 inhibitor, a dose the immune checkpoint inhibitor, and a dose of the bispecific antibody construct may be concurrently administered.

In some embodiments, a dose of the bispecific antibody molecule, a dose of the VEGFR-2 inhibitor and/or a dose the immune checkpoint inhibitor are administered as split-day dosing on two consecutive days.

According to some embodiments, the first dose of the VEGFR-2 inhibitor, the first dose of the bispecific antibody molecule, and/or the first dose of the immune checkpoint inhibitor, during the first cycle are administered as split-day dosing on two consecutive days.

In some embodiments a lead-in dose of the bispecific antibody construct may be administered to the subject about 5 to about 8 days or about 6 to about 7 days prior to a first circle of administration.

In some embodiments the lead-in dose may be in the range of about about 400 to about 550 mg, about 420 to about 530 mg, about 450 to about 500 mg, or about 480 mg.

For example, the lead-in dose may be administered as a split-dose on two consecutive days.

According to some embodiments, the bispecific antibody construct, the VEGFR-2 inhibitor, and/or the immune checkpoint inhibitor are administered intravenously.

In some embodiments, administering is carried out for a period of time in the range of 2 weeks to 2 years, or 1 month to 18 months, or 3 months to 12 months.

It is envisaged that the method may comprise assessing tumor regression after each cycle of treatment.

In some embodiments, the treatment cycle comprises treatment days spread evenly across the treatment cycle. For example, if the treatment cycle is 6 weeks long (or thereabouts), in some cases the treatment days occur every 1 week (or thereabouts). In some embodiments, the treatment cycle comprises administration of the bispecific antibody construct, the VEGFR-2 inhibitor, and/or the immune checkpoint inhibitor on each treatment day. In some embodiments, the treatment cycle comprises administration of the bispecific antibody construct, the VEGFR-2 inhibitor, and/or the immune checkpoint inhibitor on a subset of the treatment days. In some embodiments, the treatment cycle comprises administration of the bispecific antibody construct, the VEGFR-2 inhibitor, and/or the immune checkpoint inhibitor on each treatment day. In some embodiments, the treatment cycle comprises administration of the bispecific antibody construct, the VEGFR-2 inhibitor, and/or the immune checkpoint inhibitor on a subset of the treatment days.

Generally, the above described methods and administration schemes include administering a therapeutically effective amount of the CD16A and EGFR targeting bispecific antibody construct, of the immune checkpoint inhibitor, and of the VEGFR-2 inhibitor. A therapeutic effective amount or dosage of the used bispecific antibody construct, of the used immune checkpoint inhibitor, and of the used VEGFR-2 inhibitor preferably results in a decrease in severity of disease symptoms, an increase in frequency or duration of disease symptom-free periods or a prevention of impairment or disability due to the disease affliction. The term "effective dose" or "effective dosage" is defined as an amount sufficient to achieve or at least partially achieve beneficial or desired results. For example, a therapeutic amount is one that achieves the desired therapeutic effect. The term "therapeutically effective dose" is defined as an amount sufficient to cure or at least partially arrest the disease and its complications in a patient already suffering from the disease. An effective amount can be administered in one or more administrations, applications or dosages. The compositions can be administered one from one or more times per day to one or more times per week, including once every other day. Amounts or doses effective for this use will depend on the therapeutic context and objectives, the severity of the disease, prior therapy, the patient's clinical history and response to the therapeutic agent, other drugs being administered concurrently, the route of administration, the size (body weight, body surface area, age and sex,) and/or condition (general health) of the patient, and the general state of the patient's own immune system. The proper dose can be adjusted according to the judgment of the attending physician such that it can be administered to the patient once or over a series of administrations, and in order to obtain the optimal therapeutic effect.

Dosage, toxicity and therapeutic efficacy of the therapeutic compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

In some embodiments of the present invention, a "therapeutically effective dose" of the bispecific antibody construct, of the immune checkpoint inhibitor, and of the VEGFR-2 inhibitor when administered in combination to a patient can be defined as the dose leading to transforming a patient suffering from a tumor disease that is low responsive to treatment with the bispecific antibody construct if administered without the VEGFR-2 inhibitor to a patient that is high responsive to treatment with said bispecific antibody construct if administered with the VEGFR-2 inhibitor and with the immune checkpoint inhibitor, as described elsewhere herein.

In some embodiments, the bispecific antibody construct may be administered to the patient in a dose range of 25 mg to 1000 mg, such as a dose of 25 mg, 50 mg, 75 mg, 100mg, 150mg, 200 mg, 250 mg, 300 mg, 350 mg, 400 mg, 450 mg, 500 mg, 550, mg, 600 mg, 650 mg, 700 mg, 750 mg, 800 mg, 850 mg, 900 mg, 950 mg or 1000mg. In preferred embodiments, the bispecific antibody construct is administered to the patient in a range of 100 mg to 600 mg, such as 100 mg, 200 mg, 250 mg, 300 mg, 350 mg, 400 mg, 450 mg, 500 mg, 550 mg or 600 mg. In even more preferred embodiments, the bispecific antibody construct is administered to the patient in a range of 150 mg to 400 mg, such as 150 mg, 175 mg, 200 mg, 225 mg, 250 mg, 275 mg, 300 mg, 325 mg, 350 mg, 375 mg or 400 mg. In even more preferred embodiments, the bispecific antibody construct is administered to the patient in a range of 250 mg to 350 mg, such as 250 mg, 260 mg, 270 mg, 280 mg, 290 mg, 300 mg, 310 mg, 320 mg, 330 mg, 340 mg or 350 mg. In equally preferred embodiments, the bispecific antibody construct is administered to the patient in a range of 100 mg to 300 mg, such as 100 mg, 125 mg, 150 mg, 175 mg, 200 mg, 225 mg, 250 mg, 275 mg or 300 mg.

The bispecific antibody construct, the immune checkpoint inhibitor and/or the VEGFR-2 inhibitor may be be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some embodiments, the bispecific antibody construct, the immune checkpoint inhibitor and/or the VEGFR-2 inhibitor may be administered by parenteral, intrapulmonary, and intranasal, and, if desired for local treatment, intralesional administration. Parenteral infusions include intramuscular, intravenous, intraarterial, intraperitoneal, or subcutaneous administration. In some embodiments, a given dose is administered by a single bolus administration of the bispecific antibody construct, the immune checkpoint inhibitor and/or the VEGFR-2 inhibitor. In some embodiments, a given dose is administered by multiple bolus administrations of the bispecific antibody construct, the immune checkpoint inhibitor and/or the VEGFR-2 inhibitor, for example, over a period of no more than 3 days, or by continuous infusion administration of the bispecific antibody construct, the immune checkpoint inhibitor and/or the VEGFR-2 inhibitor.

If the pharmaceutical composition comprising the bispecific antibody construct, the immune checkpoint inhibitor and/or the VEGFR-2 inhibitor has been lyophilized, the lyophilized material is first reconstituted in an appropriate liquid prior to administration. The lyophilized material may be reconstituted in, e.g., bacteriostatic water for injection (BWFI), physiological saline, phosphate buffered saline (PBS), or the same formulation the protein had been in prior to lyophilization.

Pharmaceutical compositions described herein may be administered using a medical device. In some embodiments, the pharmaceutical compositions are administered intravenously via syringe. Examples of medical devices for administering pharmaceutical compositions are described in U.S. Patent Nos. 4,475,196; 4,439,196; 4,447,224; 4,447, 233; 4,486,194; 4,487,603; 4,596,556; 4,790,824; 4,941,880; 5,064,413; 5,312,335; 5,312,335; 5,383,851; and 5,399,163.

According to some embodiments, treating may result in about 5% (size) or greater shrinkage of tumor, about 10% or greater shrinkage of tumor, about 20% or greater shrinkage of tumor, about 30% or greater shrinkage of tumor, or about 50% or greater shrinkage of tumor.

In some embodiments, treating may reduce the number of regulatory T cells in the tumor microenvironment as compared to a treatment regimen comprising the administration of the bispecific antibody molecule, but without the administration of a VEGFR-2 inhibitor and the immune checkpoint inhibitor.

In some embodiments, treating may inhibit, reduce, or revert the transformation of a tumor microenvironment to an immune-suppressive tumor-microenvironment as compared to a treatment regimen comprising the administration of the bispecific antibody molecule, but without the administration of a VEGFR-2 inhibitor and an immune checkpoint inhibitor.

It is further envisaged that treating may inhibit, reduce, or revert neovascularization as compared to a treatment regimen comprising the administration of the bispecific antibody molecule, but without the administration of a VEGFR-2 inhibitor and the immune checkpoint inhibitor.

In accordance with a further aspect of the invention, there is also provided a (pharmaceutical) composition comprising a VEGFR-2 inhibitor, a bispecific antibody construct that comprises at least one first binding domain capable of specifically binding CD16A and at least one second binding domain capable of specifically binding EGFR, and optionally an immune checkpoint inhibitor.

In some embodiments, the VEGFR-2 inhibitor may be present in an amount (by weight) that is in the range of about 40 to about 60 times lower than an amount of the bispecific antibody construct.

For example, the composition may be in liquid form and configured for IV administration.

According to some embodiments, the composition may comprise one or more excipients.

It is contemplated that the composition may be for use in in a method of treating a tumor in a subject, such as in any one of the methods as herein described.

In accordance with a further aspect, there is also provided a combination comprising a VEGFR-2 inhibitor, a bispecific antibody construct that comprises at least one first binding domain capable of specifically binding CD16A and at least one second binding domain capable of specifically binding EGFR, and optionally an immune checkpoint inhibitor.

It is envisaged that the combination may be used in a method of treating a tumor in a subject, such as in any one of the methods as herein described.

According to a further aspect there is also provided herein a VEGFR-2 inhibitor or a combination of a VEGFR-2 inhibitor, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the increase of regulatory T cells in a tumor microenvironment associated with the treatment of a tumor with a bispecific antibody construct and optionally an immune checkpoint inhibitor, such as for use in any one of the methods as herein described.

According to a further aspect, there is also provided a VEGFR-2 inhibitor or a combination of a VEGFR-2 inhibitor, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the transformation of a tumor microenvironment to an immune-suppressive tumor-microenvironment associated with the treatment of a tumor with a bispecific antibody construct and optionally an immune checkpoint inhibitor, such as for use in any one of the methods as herein described.

According to a further aspect, the invention also provides a VEGFR-2 inhibitor or a combination of a VEGFR-2 inhibitor, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting neovascularization associated with the treatment of a tumor with a bispecific antibody construct and optionally an immune checkpoint inhibitor, such as for use in any one of the methods as herein described.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 10%, preferably within 5%, more preferably within 2%, even more preferably within 1% of a given value or range (plus (+) or minus (-)). It includes, however, also the concrete number, e.g., about 20 includes 20.

The term "less than" or "greater than" includes the concrete number. For example, less than 20 means less than or equal to. Similarly, more than or greater than means more than or equal to, or greater than or equal to, respectively.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein, any of the terms "comprising", "consisting essentially of" and "consisting of" may be replaced with either of the other two terms. For example, the disclosure of the term "comprising" includes the disclosure of the terms "consisting essentially of" as well as the disclosure of the term "consisting of".

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.
A better understanding of the present invention and of its advantages will be obtained from the following examples, offered for illustrative purposes only. The examples are not intended to limit the scope of the present invention in any way.
The invention is further characterized by the following items.
Item 1. A bispecific antibody construct and/or a VEGFR-2 inhibitor and/or an immune checkpoint inhibitor for use in a method of treating a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct, the VEGFR-2 inhibitor, and an immune checkpoint inhibitor.
Item 2. The bispecific antibody construct and/or a VEGFR-2 inhibitor and/or an immune checkpoint inhibitor for the use according to item 1, wherein the tumor is a solid tumor. Item 3. The bispecific antibody construct and/or a VEGFR-2 inhibitor and/or an immune checkpoint inhibitor for the use according to item 1 or 2, wherein the tumor characterized by epidermal growth factor receptor (EGFR) overexpression on tumor cells.
Item 4. The bispecific antibody construct and/or a VEGFR-2 inhibitor and/or an immune checkpoint inhibitor for the use according to any one of the foregoing items, wherein the tumor is an EGFR-positive tumor.
Item 5. The bispecific antibody construct and/or a VEGFR-2 inhibitor and/or an immune checkpoint inhibitor for the use according to any one of the foregoing items, wherein the tumor is selected from the group consisting of lung cancer, colorectal cancer, liver cancer, brain cancer, kidney/renal cancer, ovarian cancer, breast cancer, squamous cell carcinoma, bladder cancer, head and neck cancer, gastric cancer, esophagus cancer, sarcoma, mesothelioma, and adenocarcinoma, optionally, non-small cell lung cancer (NSCLC), hepatocellular carcinoma (HCC), glioblastoma (GBM), Clear Cell Renal Cell Carcinoma (ccRCC), Triple-negative breast cancer (TNBC), squamous cervical cancer, and squamous cell carcinoma of head and neck (SCCHN).
Item 6. The bispecific antibody construct and/or a VEGFR-2 inhibitor and/or an immune checkpoint inhibitor for the use according to any one of the foregoing items, wherein the tumor is lung cancer.
Item 7. The bispecific antibody construct and/or a VEGFR-2 inhibitor and/or an immune checkpoint inhibitor for the use according to item 6, wherein the tumor is non-small cell lung cancer (NSCLC).
Item 8. The bispecific antibody construct and/or a VEGFR-2 inhibitor and/or an immune checkpoint inhibitor for the use according to any one of the foregoing items, wherein the first binding domain binds to an epitope on CD16A which is C-terminal to the physiological Fcy receptor binding domain, said epitope preferably comprising Y158 of SEQ ID NO: 1.
Item 9. The bispecific antibody construct and/or a VEGFR-2 inhibitor and/or an immune checkpoint inhibitor for the use according to any one of the foregoing items, wherein the first binding domain comprises
   (i) a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13);
   (ii) a VH region comprising CDR-H1 sequence GYTFTSYY (SEQ ID NO: 17), CDR-H2 sequence IEPMYGST (SEQ ID NO: 18), and CDR-H3 sequence ARGSAYYYDFADY (SEQ ID NO: 19), and a VL region comprising CDR-L1 sequence NIGSKN (SEQ ID NO: 20), CDR-L2 sequence QDN, and CDR-L3 sequence QVWDNYSVL (SEQ ID NO: 21);
   (iii) a VH region comprising CDR-H1 sequence GYTFTNYY (SEQ ID NO: 25), CDR-H2 sequence INPSGGVT (SEQ ID NO: 26), and CDR-H3 sequence ARGSAYYYDFADY (SEQ ID NO: 27), and a VL region comprising CDR-L1 sequence NIGSKS (SEQ ID NO: 28), CDR-L2 sequence QDK, and CDR-L3 sequence QVWDDYIVL (SEQ ID NO: 29);
   (iv) a VH region comprising CDR-H1 sequence SYYMH (SEQ ID NO: 32), CDR-H2 sequence AIEPRYGSTSYAQKFQG (SEQ ID NO: 33), and CDR-H3 sequence GSAYYYDFADY (SEQ ID NO: 34), and a VL region comprising CDR-L1 sequence GGHNIGSKNVH (SEQ ID NO: 35), CDR-L2 sequence QDNKRPS (SEQ ID NO: 36), and CDR-L3 sequence QVWDNYNVL (SEQ ID NO: 37); or
   (v) a VH region comprising CDR-H1 sequence NYYMQ (SEQ ID NO: 38), CDR-H2 sequence IINPSGGVTSYAQKFQG (SEQ ID NO: 39), and CDR-H3 sequence GSAYYYDFADY (SEQ ID NO: 40), and a VL region comprising CDR-L1 sequence GGNNIGSKSVH (SEQ ID NO: 41), CDR-L2 sequence QDKKRPS (SEQ ID NO: 42), and a CDR-L3 sequence QVWDDYIVL (SEQ ID NO: 43).
Item 10. The bispecific antibody construct and/or a VEGFR-2 inhibitor and/or an immune checkpoint inhibitor for the use according to any one of items 1 to 8, wherein the first binding domain comprises a
   (i) a VH region having at least 80%, at least 85%, at least 90%, at least 95%, at least 98%, or at least 99% sequence identity to SEQ ID NO: 14 and a VL region having at least 80%, at least 85%, at least 90%, at least 95%, at least 98%, or at least 99% sequence identity to SEQ ID NO: 15;
   (ii) a VH region having at least 80%, at least 85%, at least 90%, at least 95%, at least 98%, or at least 99% sequence identity to SEQ ID NO: 22 and a VL region having at least 80%, at least 85%, at least 90%, at least 95%, at least 98%, or at least 99% sequence identity to SEQ ID NO: 23;
   (iii) a VH region having at least 80%, at least 85%, at least 90%, at least 95%, at least 98%, or at least 99% sequence identity to SEQ ID NO: 30 and a VL region having at least 80%, at least 85%, at least 90%, at least 95%, at least 98%, or at least 99% sequence identity to SEQ ID NO: 31;
   (iv) a VH region having at least 80%, at least 85%, at least 90%, at least 95%, at least 98%, or at least 99% sequence identity to SEQ ID NO: 44 and a VL region having at least 80%, at least 85%, at least 90%, at least 95%, at least 98%, or at least 99% sequence identity to SEQ ID NO: 45;
   (v) a VH region having at least 80%, at least 85%, at least 90%, at least 95%, at least 98%, or at least 99% sequence identity to SEQ ID NO: 46 and a VL region having at least 80%, at least 85%, at least 90%, at least 95%, at least 98%, or at least 99% sequence identity to SEQ ID NO: 47;
   (vi) a VH region having at least 80%, at least 85%, at least 90%, at least 95%, at least 98%, or at least 99% sequence identity to SEQ ID NO: 48 and a VL region having at least 80%, at least 85%, at least 90%, at least 95%, at least 98%, or at least 99% sequence identity to SEQ ID NO: 49;
   (vii) a VH region having at least 80%, at least 85%, at least 90%, at least 95%, at least 98%, or at least 99% sequence identity to SEQ ID NO: 50 and a VL region having at least 80%, at least 85%, at least 90%, at least 95%, at least 98%, or at least 99% sequence identity to SEQ ID NO: 51;
   (viii) a VH region having at least 80%, at least 85%, at least 90%, at least 95%, at least 98%, or at least 99% sequence identity to SEQ ID NO: 52 and a VL region having at least 80%, at least 85%, at least 90%, at least 95%, at least 98%, or at least 99% sequence identity to SEQ ID NO: 53; or
   (ix) a VH region having at least 80%, at least 85%, at least 90%, at least 95%, at least 98%, or at least 99% sequence identity to SEQ ID NO: 54 and a VL region having at least 80%, at least 85%, at least 90%, at least 95%, at least 98%, or at least 99% sequence identity to SEQ ID NO: 55.
Item 11. The bispecific antibody construct and/or a VEGFR-2 inhibitor and/or an immune checkpoint inhibitor for the use according to any one of items 1 to 8, wherein the first binding domain comprises a
   (i) a VH region as depicted in SEQ ID NO: 14 and a VL region as depicted in SEQ ID NO: 15;
   (ii) a VH region as depicted in SEQ ID NO: 22 and a VL region as depicted in SEQ ID NO: 23;
   (iii) a VH region as depicted in SEQ ID NO: 30 and a VL region as depicted in SEQ ID NO: 31;
   (iv) a VH region as depicted in SEQ ID NO: 44 and a VL region as depicted in SEQ ID NO: 45;
   (v) a VH region as depicted in SEQ ID NO: 46 and a VL region as depicted in SEQ ID NO: 47;
   (vi) a VH region as depicted in SEQ ID NO: 48 and a VL region as depicted in SEQ ID NO: 49;
   (vii) a VH region as depicted in SEQ ID NO: 53 and a VL region as depicted in SEQ ID NO: 50;
   (viii) a VH region as depicted in SEQ ID NO: 55 and a VL region as depicted in SEQ ID NO: 51; or
   (ix) a VH region as depicted in SEQ ID NO: 52 and a VL region as depicted in SEQ ID NO: 53.
Item 12. The bispecific antibody construct and/or a VEGFR-2 inhibitor and/or an immune checkpoint inhibitor for the use according to any one of the foregoing items, wherein the first binding domain is a variable domain (Fv), a single chain Fv (scFv), a Fab, a single chain diabody (scDb), a diabody (Db) or a double Fab.
Item 13. The bispecific antibody construct and/or a VEGFR-2 inhibitor and/or an immune checkpoint inhibitor for the use according to any one of the foregoing items, wherein the second binding domain comprises a VH and a VL domain of an antibody.
Item 14. The bispecific antibody construct and/or a VEGFR-2 inhibitor and/or an immune checkpoint inhibitor for the use according to any one of the foregoing items, wherein the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).
Item 15. The bispecific antibody construct and/or a VEGFR-2 inhibitor and/or an immune checkpoint inhibitor for the use according to any one of the foregoing items, wherein the second binding domain comprises a VH region having at least 80%, at least 85%, at least 90%, at least 95%, at least 98%, or at least 99% sequence identity to SEQ ID NO: 61 and a VL region having at least 80%, at least 85%, at least 90%, at least 95%, at least 98%, or at least 99% sequence identity to SEQ ID NO: 62.
Item 16. The bispecific antibody construct and/or a VEGFR-2 inhibitor and/or an immune checkpoint inhibitor for the use according to any one of the foregoing items, wherein the second binding domain comprises a VH region as depicted in SEQ ID NO: 61 and a VL region as depicted in SEQ ID NO: 62.
Item 17. The bispecific antibody construct and/or a VEGFR-2 inhibitor and/or an immune checkpoint inhibitor for the use according to any one of the foregoing items, wherein the second binding domain is a variable domain (Fv), a single chain Fv (scFv), a Fab, a single chain diabody (scDb), a diabody (Db) or a double Fab.
Item 18. The bispecific antibody construct and/or a VEGFR-2 inhibitor and/or an immune checkpoint inhibitor for the use according to any one of the foregoing items, wherein the BISPECIFIC ANTIBODY CONSTRUCT binds to a target cell and an immune effector cell simultaneously.
Item 19. The bispecific antibody construct and/or a VEGFR-2 inhibitor and/or an immune checkpoint inhibitor for the use according to any one of the foregoing items, wherein the BISPECIFIC ANTIBODY CONSTRUCT further comprises a third domain comprising a half-life extension domain.
Item 20. The bispecific antibody construct and/or a VEGFR-2 inhibitor and/or an immune checkpoint inhibitor for the use according to item 19, wherein said half-life extension domain comprises a CH2 domain, wherein the Fcγ receptor binding domain is silenced.
Item 21. The bispecific antibody construct and/or a VEGFR-2 inhibitor for the use according to any one of items 19 or 20, wherein said half-life extension domain comprises a CH3 domain.
Item 22. The bispecific antibody construct and/or a VEGFR-2 inhibitor and/or an immune checkpoint inhibitor for the use according to any one of the foregoing items, wherein the BISPECIFIC ANTIBODY CONSTRUCT comprise at least one hinge domain and a CH3 domain fused to a CH2 domain in an amino to carboxyl order in the order hinge domain - CH2 domain - CH3 domain.
Item 23. The bispecific antibody construct and/or a VEGFR-2 inhibitor and/or an immune checkpoint inhibitor for the use according to any one of the foregoing items, wherein the antibody construct comprises at least two of the hinge domain - CH2 domain - CH3 domain elements.
Item 24. The bispecific antibody construct and/or a VEGFR-2 inhibitor and/or an immune checkpoint inhibitor for the use according to any one of the foregoing items, wherein the second binding domain is fused to the C terminus of a CH3 domain and the first binding domain is fused to the N terminus of a hinge region.
Item 25. The bispecific antibody construct and/or a VEGFR-2 inhibitor and/or an immune checkpoint inhibitor for the use according to any one of the foregoing items, wherein the antibody construct is bivalent for the first binding domain and bivalent for the second binding domain.
Item 26. The bispecific antibody construct and/or a VEGFR-2 inhibitor and/or an immune checkpoint inhibitor for the use according to any one of the foregoing items, wherein
   (a) the first binding domain comprises a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13); and
   (b) the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60).
Item 27. The bispecific antibody construct and/or a VEGFR-2 inhibitor and/or an immune checkpoint inhibitor for the use according to any one of the foregoing items, wherein
   (a) the first binding domain comprises a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13), wherein said first binding domain is a Fab;
   (b) the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60), wherein said second binding domain is a scFv; and
   (c) the third binding domain comprises two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.
Item 28. The bispecific antibody construct and/or a VEGFR-2 inhibitor and/or an immune checkpoint inhibitor for the use according to any one of the foregoing items, wherein
   (a) the first binding domain comprises a VH region as depicted in SEQ ID NO: 14 and a VL region as depicted in SEQ ID NO: 15; and
   (b) the second binding domain comprises a VH region as depicted in SEQ ID NO: 61 and a VL region as depicted in SEQ ID NO: 62.
Item 29. The bispecific antibody construct and/or a VEGFR-2 inhibitor and/or an immune checkpoint inhibitor for the use according to any one of the foregoing items, wherein
   (a) the first binding domain comprises a VH region as depicted in SEQ ID NO: 14 and a VL region as depicted in SEQ ID NO: 15, wherein said first binding domain is a Fab;
   (b) the second binding domain comprises a VH region as depicted in SEQ ID NO: 61 and a VL region as depicted in SEQ ID NO: 62, wherein said second binding domain is a scFv; and
   (c) the third binding domain comprises two of the hinge domain - CH2 domain - CH3 domain elements, preferably as depicted in SEQ ID NO: 69; wherein the second binding domain is fused to the C terminus of a CH3 domain of the third domain and the first binding domain is fused to the N terminus of a hinge region of the third domain.
Item 30. The bispecific antibody construct and/or a VEGFR-2 inhibitor and/or an immune checkpoint inhibitor for the use according to any one of the foregoing items, wherein the antibody construct comprises or consists of an amino acid sequence having at least 80%, at least 85%, at least 90%, at least 95%, at least 98%, or at least 99% sequence identity to the amino acid sequences set forth in SEQ ID NOs: 63 and 64, SEQ ID NOs: 65 and 66; SEQ ID NO: 67; or SEQ ID NO: 68.
Item 31. The bispecific antibody construct and/or a VEGFR-2 inhibitor and/or an immune checkpoint inhibitor for the use according to any one of the foregoing items, wherein the antibody construct comprises or consists of an amino acid sequence set forth in SEQ ID NOs: 63 and 64, SEQ ID NOs: 65 and 66; SEQ ID NO: 67; or SEQ ID NO: 68.
Item 32. The bispecific antibody construct and/or a VEGFR-2 inhibitor and/or an immune checkpoint inhibitor for the use according to any one of the foregoing items, wherein the bispecific antibody construct is AFM24.
Item 33. The bispecific antibody construct and/or a VEGFR-2 inhibitor and/or an immune checkpoint inhibitor for the use according to any one of the foregoing items, wherein the at least one first binding domain of the bispecific antibody construct comprises means for specifically binding CD16A and the at least one second binding domain of the bispecific antibody construct comprises means for specifically binding EGFR.
Item 34. The bispecific antibody construct and/or a VEGFR-2 inhibitor and/or an immune checkpoint inhibitor for the use according to any one of the foregoing items,, wherein the bispecific antibody construct comprises means for specifically binding CD16A and EGFR.
Item 35. The bispecific antibody construct and/or a VEGFR-2 inhibitor and/or an immune checkpoint inhibitor for the use according to any one of the foregoing items, wherein the VEGFR-2 inhibitor is an anti-VEGFR-2 antibody.
Item 36. The bispecific antibody construct and/or a VEGFR-2 inhibitor and/or an immune checkpoint inhibitor for the use according to any one of the foregoing items, wherein the VEGFR-2 inhibitor is selected from the group consisting of ramucirumab, bevacizumab, and aflibercept.
Item 37. The bispecific antibody construct and/or a VEGFR-2 inhibitor and/or an immune checkpoint inhibitor for the use according to item 36, wherein the VEGFR-2 inhibitor is ramucirumab.
Item 38. The bispecific antibody construct and/or a VEGFR-2 inhibitor and/or an immune checkpoint inhibitor for the use according to any one of the foregoing items, wherein the immune checkpoint inhibitor is a PD-L1 or PD-1 inhibitor.
Item 39. The bispecific antibody construct and/or a VEGFR-2 inhibitor and/or an immune checkpoint inhibitor for the use according to any one of the foregoing items, wherein the immune checkpoint inhibitor is an anti-PD-1 antibody or an anti-PD-L1 antibody.
Item 40. The bispecific antibody construct and/or a VEGFR-2 inhibitor and/or an immune checkpoint inhibitor for the use according to any one of the foregoing items, wherein the immune checkpoint inhibitor is an anti-PD-L1 antibody.
Item 41. The bispecific antibody construct and/or a VEGFR-2 inhibitor and/or an immune checkpoint inhibitor for the use according to any one of the foregoing items, wherein the immune checkpoint inhibitor is selected from the group consisting of nivolumab atezolizumab, pembrolizumab, cemiplimab, H4H7798N, dostarlimab, durvalumab, pidilizumab, MED10608, BI 754091, PF-0680159, spartalizumab, camrelizumab, JNJ-63723283, MCLA-134, H2M8314N, avelumab, NMX-1105, LY3300054, FAZ053, STI-1014, CX-072, KN035, and CK-301, preferably wherein the immune checkpoint inhibitor is nivolumab, atezolizumab or pembrolizumab, more preferably wherein the checkpoint inhibitor is atezolizumab.
Item 42. The bispecific antibody construct and/or a VEGFR-2 inhibitor and/or an immune checkpoint inhibitor for the use according to item 41, wherein the immune checkpoint inhibitor is atezolizumab.
Item 43. The bispecific antibody construct and/or a VEGFR-2 inhibitor and/or an immune checkpoint inhibitor for the use according to any one of the foregoing items, wherein the immune checkpoint inhibitor is an antibody comprising means for specifically binding PD-L1.
Item 44. The bispecific antibody construct and/or a VEGFR-2 inhibitor and/or an immune checkpoint inhibitor for the use according to any one of the foregoing items, wherein the bispecific antibody construct is administered to the subject once every about 6 to about 8 days, or once every about 7 days.
Item 45. The bispecific antibody construct and/or a VEGFR-2 inhibitor and/or an immune checkpoint inhibitor for the use according to any one of the foregoing items, wherein the bispecific antibody construct is administered at a dose in the range of about about 300 to about 800 mg, about 400 to about 550 mg, about 420 to about 530 mg, about 450 to about 500 mg, or about 480 mg.
Item 46. The bispecific antibody construct and/or a VEGFR-2 inhibitor and/or an immune checkpoint inhibitor for the use according to any one of the foregoing items, wherein the VEGFR-2 inhibitor is administered to the subject once every about 1 to about 3 weeks, once every about 2 weeks, once every about 12 to about 16 days, once every about 13 to about 15 days, or once every about 14 days.
Item 47. The bispecific antibody construct and/or a VEGFR-2 inhibitor and/or an immune checkpoint inhibitor for the use according to any one of the foregoing items, wherein the VEGFR-2 inhibitor is administered at a dose in the range of about 5 to about 15 mg, about 8 to about 12 mg, about 9 to about 11 mg, or about 10 mg.
Item 48. The bispecific antibody construct and/or a VEGFR-2 inhibitor and/or an immune checkpoint inhibitor for the use according to any one of the foregoing items, wherein the immune checkpoint inhibitor is administered to the subject once every about 1 to about 3 weeks, once every about 2 weeks, once every about 12 to about 16 days, once every about 13 to about 15 days, or once every about 14 days.
Item 49. The bispecific antibody construct and/or a VEGFR-2 inhibitor and/or an immune checkpoint inhibitor for the use according to any one of the foregoing items, wherein the immune checkpoint inhibitor is administered at a dose in the range of about 500 to about 1200 mg, about 700 to about 1000 mg, about 800 to about 900 mg, or about 840 mg.
Item 50. The bispecific antibody construct and/or a VEGFR-2 inhibitor and/or an immune checkpoint inhibitor for the use according to any one of the foregoing items, wherein a cycle of administering consists of administering the bispecific antibody construct molecule 4 times, administering the VEGFR-2 inhibitor 2 times, and administering the immune checkpoint inhibitor 2 times, and treatment of the subject is carried out for at least one cycle.
Item 51. The bispecific antibody construct and/or a VEGFR-2 inhibitor and/or an immune checkpoint inhibitor for the use according to any one of the foregoing items, wherein a cycle of administering is about 4 weeks.
Item 52. The bispecific antibody construct and/or a VEGFR-2 inhibitor and/or an immune checkpoint inhibitor for the use according to any one of the foregoing items, wherein administering of the VEGFR-2 inhibitor and administering of the immune checkpoint inhibitor are at the same time or about at the same time.
Item 53. The bispecific antibody construct and/or a VEGFR-2 inhibitor and/or an immune checkpoint inhibitor for the use according to any one of the foregoing items, wherein the VEGFR-2 inhibitor is administered in the first week and the third week of each cycle.
Item 54. The bispecific antibody construct and/or a VEGFR-2 inhibitor and/or an immune checkpoint inhibitor for the use according to any one of the foregoing items, wherein a dose the VEGFR-2 inhibitor and a dose of the bispecific antibody construct are administered to the subject at the same time, or about the same time.
Item 55. The bispecific antibody construct and/or a VEGFR-2 inhibitor and/or an immune checkpoint inhibitor for the use according to any one of the foregoing items, wherein a dose the VEGFR-2 inhibitor is concurrently administered with a dose of the bispecific antibody construct.
Item 56. The bispecific antibody construct and/or a VEGFR-2 inhibitor and/or an immune checkpoint inhibitor for the use according to any one of the foregoing items, wherein a dose of the bispecific antibody molecule and/or a dose the VEGFR-2 inhibitor are administered as split-day dosing on two consecutive days.
Item 57. The bispecific antibody construct and/or a VEGFR-2 inhibitor and/or an immune checkpoint inhibitor for the use according to any one of the foregoing items, wherein the first dose of the bispecific antibody molecule and the first dose of VEGFR-2 inhibitor during the first cycle are administered as split-day dosing on two consecutive days.
Item 58. The bispecific antibody construct and/or a VEGFR-2 inhibitor and/or an immune checkpoint inhibitor for the use according to any one of the foregoing items, wherein the immune checkpoint inhibitor is administered in the first and third week of each cycle.
Item 59. The bispecific antibody construct and/or a VEGFR-2 inhibitor and/or an immune checkpoint inhibitor for the use according to any one of the foregoing items, wherein a dose the immune checkpoint inhibitor and a dose of the bispecific antibody construct are administered to the subject at the same time, or about the same time.
Item 60. The bispecific antibody construct and/or a VEGFR-2 inhibitor and/or an immune checkpoint inhibitor for the use according to any one of the foregoing items, wherein a dose the immune checkpoint inhibitor is concurrently administered with a dose of the bispecific antibody construct.
Item 61. The bispecific antibody construct and/or a VEGFR-2 inhibitor and/or an immune checkpoint inhibitor for the use according to any one of the foregoing items, wherein a dose of the bispecific antibody molecule and/or a dose the immune checkpoint inhibitor are administered as split-day dosing on two consecutive days.
Item 62. The bispecific antibody construct and/or a VEGFR-2 inhibitor and/or an immune checkpoint inhibitor for the use according to any one of the foregoing items, wherein the first dose of the bispecific antibody molecule and the first dose of immune checkpoint inhibitor during the first cycle are administered as split-day dosing on two consecutive days.
Item 63. The bispecific antibody construct and/or a VEGFR-2 inhibitor and/or an immune checkpoint inhibitor for the use according to any one of the foregoing items, wherein the VEGFR-2 inhibitor and the immune checkpoint inhibitor are administered in the first and third week of each cycle.
Item 64. The bispecific antibody construct and/or a VEGFR-2 inhibitor and/or an immune checkpoint inhibitor for the use according to any one of the foregoing items, wherein a dose the VEGFR-2 inhibitor, a dose of the immune checkpoint inhibitor and a dose of the bispecific antibody construct are administered to the subject at the same time, or about the same time.
Item 65. The bispecific antibody construct and/or a VEGFR-2 inhibitor and/or an immune checkpoint inhibitor for the use according to any one of the foregoing items, wherein a dose of the VEGFR-2 inhibitor, a dose of the immune checkpoint inhibitor, and a dose of the bispecific antibody construct are concurrently administered.
Item 66. The bispecific antibody construct and/or a VEGFR-2 inhibitor and/or an immune checkpoint inhibitor for the use according to any one of the foregoing items, wherein a dose of the bispecific antibody molecule, a dose of the VEGFR-2 inhibitor and/or a dose the immune checkpoint inhibitor are administered as split-day dosing on two consecutive days.
Item 67. The bispecific antibody construct and/or a VEGFR-2 inhibitor and/or an immune checkpoint inhibitor for the use according to any one of the foregoing items, wherein the first dose of the VEGFR-2 inhibitor, the first dose of the bispecific antibody molecule, and the first dose of immune checkpoint inhibitor, during the first cycle are administered as split-day dosing on two consecutive days.
Item 68. The bispecific antibody construct and/or a VEGFR-2 inhibitor and/or an immune checkpoint inhibitor for the use according to any one of the foregoing items, wherein a lead-in dose of the bispecific antibody construct is administered to the subject about 5 to about 8 days or about 6 to about 7 days prior to a first circle of administration.
Item 69. The bispecific antibody construct and/or a VEGFR-2 inhibitor and/or an immune checkpoint inhibitor for the use according to item 68, wherein the lead-in dose is in the range of about about 400 to about 550 mg, about 420 to about 530 mg, about 450 to about 500 mg, or about 480 mg.
Item 70. The bispecific antibody construct and/or a VEGFR-2 inhibitor and/or an immune checkpoint inhibitor for the use according to items 68 or 69, wherein the lead-in dose is administered as a split-dose on two consecutive days.
Item 71. The bispecific antibody construct and/or a VEGFR-2 inhibitor and/or an immune checkpoint inhibitor for the use according to any one of the foregoing items, wherein the bispecific antibody construct, the VEGFR-2 inhibitor, and/or the immune checkpoint inhibitor are administered intravenously.
Item 72. The bispecific antibody construct and/or a VEGFR-2 inhibitor and/or an immune checkpoint inhibitor for the use according to any one of the foregoing items, wherein administering is carried out for a period of time in the range of 2 weeks to 2 years, or 1 month to 18 months, or 3 months to 12 months.
Item 73. The bispecific antibody construct and/or a VEGFR-2 inhibitor and/or an immune checkpoint inhibitor for the use according to any one of the foregoing items, wherein the method comprises assessing tumor regression after each cycle of treatment.
Item 74. The bispecific antibody construct and/or a VEGFR-2 inhibitor and/or an immune checkpoint inhibitor for the use according to any one of the foregoing items, wherein treating results in about 5% (size) or greater shrinkage of tumor, about 10% or greater shrinkage of tumor, about 20% or greater shrinkage of tumor, about 30% or greater shrinkage of tumor, or about 50% or greater shrinkage of tumor.
Item 75. The bispecific antibody construct and/or a VEGFR-2 inhibitor and/or an immune checkpoint inhibitor for the use according to any one of the foregoing items, wherein treating reduces the number of regulatory T cells in the tumor microenvironment as compared to a treatment regimen comprising the administration of the bispecific antibody molecule and the immune checkpoint inhibitor, but without the administration of a VEGFR-2 inhibitor.
Item 76. The bispecific antibody construct and/or a VEGFR-2 inhibitor and/or an immune checkpoint inhibitor for the use according to any one of the foregoing items, wherein treating inhibits, reduces, or reverts the transformation of a tumor microenvironment to an immune-suppressive tumor-microenvironment as compared to a treatment regimen comprising the administration of the bispecific antibody molecule and the immune checkpoint inhibitor, but without the administration of a VEGFR-2 inhibitor.
Item 77. The bispecific antibody construct and/or a VEGFR-2 inhibitor and/or an immune checkpoint inhibitor for the use according to any one of the foregoing items, wherein treating inhibits, reduces, or reverts neovascularization as compared to a treatment regimen comprising the administration of the bispecific antibody molecule and the immune checkpoint inhibitor, but without the administration of a VEGFR-2 inhibitor.
Item 78. A composition comprising a VEGFR-2 inhibitor and a bispecific antibody construct that comprises at least one first binding domain capable of specifically binding CD16A and at least one second binding domain capable of specifically binding EGFR.
Item 79. The composition according to item 78, wherein the VEGFR-2 inhibitor is present in an amount (by weight) that is in the range of about 40 to about 60 times lower than an amount of the bispecific antibody construct.
Item 80. The composition according to item 78 or 79, wherein the composition is in liquid form and configured for IV administration.
Item 81. The composition according to any one of items 78 - 80, comprising one or more excipients.
Item 82. The composition according to any one of items 78 - 81, for use in in a method of treating a tumor in a subject.
Item 83. The composition according to item 82, wherein the use is in a method as defined in any one of items 1 to 77.
Item 84. A combination comprising a VEGFR-2 inhibitor, a bispecific antibody construct that comprises at least one first binding domain capable of specifically binding CD16A and at least one second binding domain capable of specifically binding EGFR, and an immune checkpoint inhibitor.
Item 85. The combination of item 84 for use in a method of treating a tumor in a subject.
Item 86. The combination according to item 85, wherein the use is in a method as defined in any one of items 1-77.
Item 87. A VEGFR-2 inhibitor or a combination of a VEGFR-2 inhibitor, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the increase of regulatory T cells in a tumor microenvironment associated with the treatment of a tumor with a bispecific antibody construct and optionally an immune checkpoint inhibitor.
Item 88. The VEGFR-2 inhibitor or the combination of a VEGFR-2 inhibitor, a bispecific antibody construct and optionally an immune checkpoint inhibitor according to item 87, wherein the use is in a method as defined in any one of items 1-77.
Item 89. A VEGFR-2 inhibitor or a combination of a VEGFR-2 inhibitor, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting the transformation of a tumor microenvironment to an immune-suppressive tumor-microenvironment associated with the treatment of a tumor with a bispecific antibody construct and optionally an immune checkpoint inhibitor.
Item 90. The VEGFR-2 inhibitor or the combination of a VEGFR-2 inhibitor, a bispecific antibody construct and optionally an immune checkpoint inhibitor of item 89, wherein the use is in a method as defined in any one of items 1-77.
Item 91. A VEGFR-2 inhibitor or a combination of a VEGFR-2 inhibitor, a bispecific antibody construct and optionally an immune checkpoint inhibitor, wherein the bispecific antibody construct comprises at least one first binding domain capable of specifically binding CD16A and at least one second binding domain capable of specifically binding EGFR, for use in a method of inhibiting, reducing, or reverting neovascularization associated with the treatment of a tumor with a bispecific antibody construct and optionally an immune checkpoint inhibitor.
Item 92. The VEGFR-2 inhibitor or the combination of a VEGFR-2 inhibitor, a bispecific antibody construct and optionally an immune checkpoint inhibitor according to item 91, wherein the use is in a method as defined in any one of items 1-77.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 10%, preferably within 5%, more preferably within 2%, even more preferably within 1% of a given value or range (plus (+) or minus (-)). It includes, however, also the concrete number, e.g., about 20 includes 20.

The term "less than" or "greater than" includes the concrete number. For example, less than 20 means less than or equal to. Similarly, more than or greater than means more than or equal to, or greater than or equal to, respectively.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms. For example, when disclosure uses the term "comprising", the disclosure also encompasses the replacement of the term "comprising" with the terms "consisting essentially of" as well as "consisting of" and *vice versa.* E.g., the term "comprising" is meant to provide explicit support also for its replacement with "consisting essentially of" and/or "consisting of", the term "consisting essentially of" is meant to provide explicit support also for its replacement with "comprising" and/or "consisting of", and the term "consisting of" is meant to provide explicit support also for its replacement with "consisting essentially of" and "comprising". The possibility to replace terms with each other is not to be understood that these terms are synonymous. For the avoidance of doubt, the term "comprising", "consisting essentially of" or "consisting of" that is explicitly recited in the respective context is the preferred term, while its replacement with any one of the other two terms is less preferred.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

A better understanding of the present invention and of its advantages will be obtained from the following examples, offered for illustrative purposes only. The examples are not intended to limit the scope of the present invention in any way.

### Examples

### Example 1: Paired tumor biopsies indicate an increase of regulatory T cells (Tregs) in the tumor microenvironment

### Methods

Needle biopsies were taken at screening and two days after the third AFM24 dose (Cycle 1 Day 24) to assess the effect of AFM24 treatment on the tumor and the tumor microenvironment.

### Gene expression profiling

Non-spatial gene expression profiling was performed to detect changes in cellular composition and signaling in tumor biopsies which can be linked to the treatment of AFM24. Tumor biopsies of patients treated with AFM24 at doses higher than 160 mg (high dose) were analyzed by gene expression profiling using the Nanostring nCounter^{®} PanCancer Immune Profiling Panel and Tumor Signaling 360^{™} Panel, respectively.

Briefly, 250 ng of total RNA quantified using the NanoDrop^{™} 2000 (Thermo Scientific), were directly hybridized (at 65 °C for 18 hours) with the nCounter^{®} PanCancer Immune Profiling Panel and the nCounter^{®} Tumor Signaling 360TM panel following manufacturer's instructions. After solution-phase hybridization between target RNA and reporter-capture probe pairs, excess probes were washed away using a two steps magnetic bead-based purification on the nCounter^{®} Prep Station. Finally, the RNA/Probe complexes were aligned and immobilized in the cartridge for data collection. The cartridge was then transferred to the nCounter^{®} Digital Analyzer for image acquisition and counts collection.

Data analysis of gene expression data was performed using NanoString nSolver^{®} software v4.0 and nCounter^{®} Advanced Analysis module v2.0.134. Quality control was done according to default settings using nSolver software. Background correction was conducted with background thresholding by calculating the mean of negative control expression plus double of the standard deviation. The expression counts were normalized using the most stable housekeeping genes using the geNorm algorithm according to default settings. Cell type scores were calculated as the geometric mean of log2 normalized expression levels of cell type-specific genes. Pathway scores have been calculated by pathway level analysis of gene expression (PLAGE) as implemented in the nCounter^{®} Advanced Analysis module. Visualization of results has been done using R v4.3.0. The assay was not validated and exploratory.

### Results

FOXP3 is a specific marker gene for Tregs and has been used to calculate a Treg-specific cell type score. This cell type score indicates an increase of Tregs in tumor biopsies in most patients upon AFM24 treatment (Figure 1.A). Cell type score calculation for Tregs based on FOXP3 expression in paired biopsies from screening and at C1D24 of patients treated with AFM24 at doses higher than 160 mg. Biopsies have been analyzed by gene expression profiling using the Nanostring nCounter^{®} PanCancer Immune Profiling Panel and Tumor Signaling 360^{™} Panel, respectively. Boxplot showing the log2 cell type score for Tregs in paired tumor biopsies. Dots represent individual patients. (n = 10) (C1D24, Cycle 1 Day 24).

The induction of an immune suppressive milieu within the tumor upon AFM24 administration has been further confirmed by an upregulation of TGFB 1 (Figure 1.B), which encodes an immune suppressive cytokine known to reduce NK cell-mediated cytotoxicity, inhibit T cell proliferation and promote the differentiation of naïve T cells into Tregs. The induction of an immune suppressive TME comes along with an increase in expression of CD56dim NK cell-associated genes (data not shown), indicating NK cell engagement in line with AFM24 MoA. Furthermore, an increase in CD56dim NK cells and CD8 T cell scores as well as cytotoxicity-associated genes could be observed suggesting a migration of innate and adaptive cytotoxic cells to the tumor (data not shown). However, the anti-tumoral effect of the cytotoxic immune cells might be antagonized by an immune suppressive TME potentially reducing AFM24 effectiveness. Specifically, Fig. 1B shows the quantification of TGFB 1 expression in paired biopsies from screening and at C1D24 of patients treated with AFM24 at doses higher than 160 mg. Biopsies have been analyzed by gene expression profiling using the Nanostring nCounter^{®} PanCancer Immune Profiling Panel and Tumor Signaling 360^{™} Panel, respectively. Boxplot showing the normalized log2 expression of TGFB 1 in paired tumor biopsies. Dots represent individual patients. (n = 10) (C1D24, Cycle 1 Day 24)

In addition, a multitude of cancer-related signaling pathways have been analyzed calculating pathway scores using pre-defined gene sets of several cancer signaling pathways. This analysis indicates an activation of the VEGF signaling cascade upon AFM24 treatment (Figure 1C), which seems to be a side effect of AFM24 immunotherapy. It has been shown that VEGF signaling plays a significant role in tumor angiogenesis and in immune suppression, including the promotion of Tregs and upregulation of immune checkpoint molecules. These mechanisms highlight the immune suppressive function of VEGF signaling and make it a target for combination therapies involving anti-VEGF and AFM24 immunotherapy to enhance anti-tumor immunity. On top of this, anti-VEGF treatment can normalize of blood vessel formation in the tumor and with that facilitate the infiltration of immune cells into the tumor. Specifically, Fig 1C shows the calculation of a pathway score for the VEGF signaling cascade in paired biopsies from screening and at C1D24 of patients treated with AFM24 at doses higher than 160 mg. Biopsies have been analyzed by gene expression profiling using the Nanostring nCounter^{®} PanCancer Immune Profiling Panel and Tumor Signaling 360^{™} Panel, respectively. The VEGF signaling pathway score is based on a pre-defined gene set as defined by Tumor Signaling 360^{™} Panel kit from Nanostring. Boxplot showing the pathways scores for VEGF signaling in paired tumor biopsies. Dots represent individual patients. (n = 10) (C1D24, Cycle 1 Day 24).

### Sequence Listing

| Seq ID NO | Description | Sequence |
|---|---|---|
| 1 | Linker L1 | GGSGGS |
| 2 | Linker L2 | GGS GGS GGS GGS GGS GGS |
| 3 | Linker L3 | GGSGGSGGSGGSGGSGGSGGS |
| 4 | Linker L4 | GGSGGSGGS |
| 5 | Linker L5 | GGGGS |
| 6 | Linker L6 | GGGGSGGGGS |
| 7 | Linker L7 | GGGGSGGGGSGGGGSGGGGS |
| 8 | Linker L8 | GGGGS GGGGS GGGGS GGGGS GGGGS GGGGS |
| 9 | CDR-H1 CD16A (LSIV21) | GYTFTSYY |
| 10 | CDR-H2 CD16A (LSIV21) | INPSGGST |
| 11 | CDR-H3 CD16A (LSIV21) | ARGSAYYYDFADY |
| 12 | CDR-L1 CD16A (LSIV21) | NIGSKN |
| | CDR-L2 CD16A (LSIV21) | QDN |
| 13 | CDR-L3 CD16A (LSIV21) | QVWDNYSVL |
| 14 | VH CD16A (LSIV21) | QVQLVQSGAEVKKPGESLKVSCKASGYTFTSYYMHWVRQAPGQGLEWMGIINPSGGSTSYA QKFQGRVTMTRDTSTSTVYMELSSLRSEDTAVYYCARGSAYYYDFADYWGQGTLVTVSS |
| 15 | VL CD16A (LSIV21) | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVL |
| 16 | scFv CD16A (LSIV21) | QVQLVQSGAEVKKPGESLKVSCKASGYTFTSYYMHWVRQAPGQGLEWMGIINPSGGSTSYA QKFQGRVTMTRDTSTSTVYMELSSLRSEDTAVYYCARGSAYYYDFADYWGQGTLVTVSSGG SGGSGGSGGSGGSGGSSYVLTQPSSVSVAPGQTATISCGGHNIGSKNVHWYQQRPGQSPVL VIYQDNKRPSGIPERFSGSNSGNTATLTISGTQAMDEADYYCQVWDNYSVLFGGGTKLTVL AAAGSHHHHHH |
| 17 | CDR-H1 CD16A (P2C-47) | GYTFTSYY |
| 18 | CDR-H2 CD16A (P2C-47) | IEPMYGST |
| 19 | CDR-H3 CD16A (P2C-47) | ARGSAYYYDFADY |
| 20 | CDR-L1 CD16A (P2C-47) | NIGSKN |
| | CDR-L2 CD16A (P2C-47) | QDN |
| 21 | CDR-L3 CD16A (P2C-47) | QVWDNYSVL |
| 22 | VH CD16A (P2C-47) | QVQLVQSGAEVKKPGESLKVSCKASGYTFTSYYMHWVRQAPGQGLEWMGAIEPMYGSTSYA QKFQGRVTMTRDTSTSTVYMELSSLRSEDTAVYYCARGSAYYYDFADYWGQGTLVTVSS |
| 23 | VL CD16A (P2C-47) | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVL |
| 24 | scFv CD16A (P2C-47) | QVQLVQSGAEVKKPGESLKVSCKASGYTFTSYYMHWVRQAPGQGLEWMGAIEPMYGSTSYA QKFQGRVTMTRDTSTSTVYMELSSLRSEDTAVYYCARGSAYYYDFADYWGQGTLVTVSSGG SGGSGGSGGSGGSGGSSYVLTQPSSVSVAPGQTATISCGGHNIGSKNVHWYQQRPGQSPVL VIYQDNKRPSGIPERFSGSNSGNTATLTISGTQAMDEADYYCQVWDNYSVLFGGGTKLTVL AAAGSHHHHHH |
| 25 | CDR-H1 CD16A (ABC1197) | GYTFTNYY |
| 26 | CDR-H2 CD16A (ABC1197) | INPSGGVT |
| 27 | CDR-H3 CD16A (ABC1197) | ARGSAYYYDFADY |
| 28 | CDR-L1 CD16A (ABC1197) | NIGSKS |
| | CDR-L2 CD16A (ABC1197) | QDK |
| 29 | CDR-L3 CD16A (ABC1197) | QVWDDYIVL |
| 30 | VH CD16A (ABC1197) | QVQLVQSGAEVKKPGESLKVSCKASGYTFTNYYMQWVRQAPGQGLEWMGIINPSGGVTSYA QKFQGRVTMTRDTSTSTVYMELSSLRSEDTAVYYCARGSAYYYDFADYWGQGTLVTVSS |
| 31 | VL CD16A (ABC1197) | SYVLTQPSSVSVAPGQTARITCGGNNIGSKSVHWYQQKPGQAPVLVIYQDKKRPSGIPERF SGSNSGNTATLTISGTQAMDEADYYCQVWDDYIVLFGGGTKLTVL |
| 32 | CDR-H1 CD16A (P2C-47var) | SYYMH |
| 33 | CDR-H2 CD16A (P2C-47var) | AIEPRYGSTSYAQKFQG |
| 34 | CDR-H3 CD16A (P2C-47var) | GSAYYYDFADY |
| 35 | CDR-L1 CD16A (P2C-47var) | GGHNIGSKNVH |
| 36 | CDR-L2 CD16A (P2C-47var) | QDNKRPS |
| 37 | CDR-L3 CD16A (P2C-47var) | QVWDNYNVL |
| 38 | CDR-H1 CD16A (ABC1197var) | NYYMQ |
| 39 | CDR-H2 CD16A (ABC1197var) | IINPSGGVTSYAQKFQG |
| 40 | CDR-H3 CD16A (ABC1197var) | GSAYYYDFADY |
| 41 | CDR-L1 CD16A (ABC1197var) | GGNNIGSKSVH |
| 42 | CDR-L2 CD16A (ABC1197var) | QDKKRPS |
| 43 | CDR-L3 CD16A (ABC1197var) | QVWDDYIVL |
| 44 | CD16A (P2C47var46) VH | EVQLVQSGAEVKKPGASVKVSCKASGYTFTSYYMHWVRQAPGQCLEWMGAIEPTYGSTSYA QKFQGRVTMTRDTSTSTVYMELSSLRSEDTAVYYCARGSAYYYDFADYWGQGTLVTVSS |
| 45 | CD16A (P2C47var46) VL | SYELTQPLSVSVALGQTARITCGGHNIGSKNVHWYQQKPGQAPVLVIYQDNKRPSGIPERF SGSNSGNTATLTISRAQAGDEADYYCQVWDNYNVLFGCGTKLTVL |
| 46 | CD16A P2C47var50 VH | QVQLVQSGAEVKKPGASVKVSCKASGYTFTSYYMHWVRQAPGQCLEWMGAIEPTYGSTSYA QKFQGRVTMTRDTSTSTVYMELSSLRSEDTAVYYCARGSAYYYDFADYWGQGTLVTVSS |
| 47 | CD16A (P2C47var50) VL | SYELTQPLSVSVALGQTARITCGGHNIGSKNVHWYQQKPGQAPVLVIYQDNKRPSGIPERF SGSNSGNTATLTISRAQAGDEADYYCQVWDNYNVLFGCGTKLTVL |
| 48 | CD16A P2C47var51 VH | QVQLVQSGAEVKKPGASVKVSCKASGYTFTSYYMHWVRQAPGQCLEWMGAIEPRYGSTSYA QKFQGRVTMTRDTSTSTVYMELSSLRSEDTAVYYCARGSAYYYDFADYWGQGTLVTVSS |
| 49 | CD16A (P2C47var51) VL | SYELTQPLSVSVALGQTARITCGGHNIGSKNVHWYQQKPGQAPVLVIYQDNKRPSGIPERF SGSNSGNTATLTISRAQAGDEADYYCQVWDNYNVLFGCGTKLTVL |
| 50 | CD16A (P2C47var52) VH | QVQLVQSGAEVKKPGASVKVSCKASGYTFTSYYMHWVRQAPGQCLEWMGAIEPTYGSTSYA QKFQGRVTMTRDTSTSTVYMELSSLRSEDTAVYYCARGSAYYYDFADYWGQGTLVTVS |
| 51 | CD16A (P2C47var52) VL | SYELTQPLSVSVALGQTARITCGGHNIGSKNVHWYQQKPGQAPVLVIYQDNKRPSGIPERF SGSNSGNTATLTISRAQAGDEADYYCQVWDNYNVLFGCGTKLTVL |
| 52 | CD16A (ABC1197var8 ) VH | QVQLVQSGAEVKKPGASVKVSCKASGYTFTNYYMQWVRQAPGQCLEWMGIINPSGGVTSYA QKFQGRVTMTRDTSTSTVYMELSSLRSEDTAVYYCARGSAYYYDFADYWGQGTLVTVSS |
| 53 | CD16A (ABC1197var8 ) VL | SYELTQPLSVSVALGQTARITCGGNNIGSKSVHWYQQKPGQAPVLVIYQDKKRPSGIPERF SGSNSGNTATLTISRAQAGDEADYYCQVWDDYIVLFGCGTKLTVL |
| 54 | CD16A ABC1197var11 VH | QVQLVQSGAEVKKPGASVKVSCKASGYTFTNYYMQWVRQAPGQCLEWMGIINPSGGVTSYA QKFQGRVTMTRDTSTSTVYMELSSLRSEDTAVYYCARGSAYYYDFADYWGQGTLVTVSSG |
| 55 | CD16A (ABC1197var8 ) VL | SYELTQPLSVSVALGQTARITCGGNNIGSKSVHWYQQKPGQAPVLVIYQDKKRPSGIPERF SGSNSGNTATLTISRAQAGDEADYYCQVWDDYIVLFGCGTKLTVL |
| 56 | CDR-H1 EGFR | GGSVSSGSYY |
| 57 | CDR-H2 EGFR | IYYSGST |
| 58 | CDR-H3 EGFR | ARNPISIPAFDI |
| 59 | CDR-L1 EGFR | NIGSKS |
| | CDR-L2 EGFR | YDS |
| 60 | CDR-L3 EGFR | QVWDTSSDHVL |
| 61 | VH EGFR | QVQLQESGPGLVKPSETLSLTCTVSGGSVSSGSYYWSWIRQPPGKGLEWIGYIYYSGSTNY NPSLKSRVTISVDTSKNQFSLKLSSVTAADTAVYYCARNPISIPAFDIWGQGTMVTVSS |
| 62 | VL EGFR | QPVLTQPPSVSVAPGKTARITCGGNNIGSKSVHWYQQKPGQAPVLVIYYDSDRPSGIPERF SGSNSGNTATLTISRVEAGDEADYYCQVWDTSSDHVLFGGGTKLTVL |
| 63 | AFM24 H-chain | QVQLVQSGAEVKKPGESLKVSCKASGYTFTSYYMHWVRQAPGQGLEWMGIINPSGGSTSYA QKFQGRVTMTRDTSTSTVYMELSSLRSEDTAVYYCARGSAYYYDFADYWGQGTLVTVSSAS TKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEFEGGPSVF LFPPKPKDTLMISRTPEVTCVVVAVSHEDPEVKFNWYVDGVEVHNAKTKPREEQYNSTYRV VSVLTVLHQDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRWQQGNVFS CSVMHEALHNHYTQKSLSLSPGGGGGSGGGGSQVQLQESGPGLVKPSETLSLTCTVSGGSV SSGSYYWSWIRQPPGKGLEWIGYIYYSGSTNYNPSLKSRVTISVDTSKNQFSLKLSSVTAA DTAVYYCARNPISIPAFDIWGQGTMVTVSSGGSGGSGGSGGSGGSGGSQPVLTQPPSVSVA PGKTARITCGGNNIGSKSVHWYQQKPGQAPVLVIYYDSDRPSGIPERFSGSNSGNTATLTI SRVEAGDEADYYCQVWDTSSDHVLFGGGTKLTVL |
| 64 | AFM24 L-chain | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVLGQPKAAPSVTLFPPSS EELQANKATLVCLISDFYPGAVTVAWKADSSPVKAGVETTTPSKQSNNKYAASSYLSLTPE QWKSHRSYSCQVTHEGSTVEKTVAPTECS |
| 65 | AFM24-2 H-chain | QVQLVQSGAEVKKPGESLKVSCKASGYTFTSYYMHWVRQAPGQGLEWMGAIEPMYGSTSYA QKFQGRVTMTRDTSTSTVYMELSSLRSEDTAVYYCARGSAYYYDFADYWGQGTLVTVSSAS TKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEFEGGPSVF LFPPKPKDTLMISRTPEVTCVVVAVSHEDPEVKFNWYVDGVEVHNAKTKPREEQYNSTYRV VSVLTVLHQDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSRWQQGNVFS CSVMHEALHNHYTQKSLSLSPGGGGGSGGGGSQVQLQESGPGLVKPSETLSLTCTVSGGSV SSGSYYWSWIRQPPGKGLEWIGYIYYSGSTNYNPSLKSRVTISVDTSKNQFSLKLSSVTAA DTAVYYCARNPISIPAFDIWGQGTMVTVSSGGSGGSGGSGGSGGSGGSQPVLTQPPSVSVA PGKTARITCGGNNIGSKSVHWYQQKPGQAPVLVIYYDSDRPSGIPERFSGSNSGNTATLTI SRVEAGDEADYYCQVWDTSSDHVLFGGGTKLTVL |
| 66 | AFM24-2 L-chain | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVLGQPKAAPSVTLFPPSS EELQANKATLVCLISDFYPGAVTVAWKADSSPVKAGVETTTPSKQSNNKYAASSYLSLTPE QWKSHRSYSCQVTHEGSTVEKTVAPTECS |
| 67 | AFM24_T | QVQLQESGPGLVKPSETLSLTCTVSGGSVSSGSYYWSWIRQPPGKGLEWIGYIYYSGSTNY NPSLKSRVTISVDTSKNQFSLKLSSVTAADTAVYYCARNPISIPAFDIWGQGTMVTVSSGG SGGSGGSSYVLTQPSSVSVAPGQTATISCGGHNIGSKNVHWYQQRPGQSPVLVIYQDNKRP SGIPERFSGSNSGNTATLTISGTQAMDEADYYCQVWDNYSVLFGGGTKLTVLGGSGGSQVQ LVQSGAEVKKPGESLKVSCKASGYTFTSYYMHWVRQAPGQGLEWMGIINPSGGSTSYAQKF QGRVTMTRDTSTSTVYMELSSLRSEDTAVYYCARGSAYYYDFADYWGQGTLVTVSSGGSGG SGGSQPVLTQPPSVSVAPGKTARITCGGNNIGSKSVHWYQQKPGQAPVLVIYYDSDRPSGI PERFSGSNSGNTATLTISRVEAGDEADYYCQVWDTSSDHVLFGGGTKLTVLAAAGSHHHHH H |
| 68 | AFM24_Bi-scFv-Fc | SYVLTQPSSVSVAPGQTATISCGGHNIGSKNVHWYQQRPGQSPVLVIYQDNKRPSGIPERF SGSNSGNTATLTISGTQAMDEADYYCQVWDNYSVLFGGGTKLTVLGQPKAAPSVTLFPPSS EELQANKATLVCLISDFYPGAVTVAWKADSSPVKAGVETTTPSKQSNNKYAASSYLSLTPE QWKSHRSYSCQVTHEGSTVEKTVAPTECSLTVDKSRWQQGNVFSCSVMHEALHNHYTQKSL SLSPGGGGGSGGGGSQVQLQESGPGLVKPSETLSLTCTVSGGSVSSGSYYWSWIRQPPGKG LEWIGYIYYSGSTNYNPSLKSRVTISVDTSKNQFSLKLSSVTAADTAVYYCARNPISIPAF DIWGQGTMVTVSSGGSGGSGGSGGSGGSGGSQPVLTQPPSVSVAPGKTARITCGGNNIGSK SVHWYQQKPGQAPVLVIYYDSDRPSGIPERFSGSNSGNTATLTISRVEAGDEADYYCQVWD TSSDHVLFGGGTKLTVL |
| 69 | Hinge - CH2 domain - CH3 domain | EPKSCDKTHTCPPCPAPEFEGGPSVFLFPPKPKDTLMISRTPEVTCVVVAVSHEDPEVKFN WYVDGVEVHNAKTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQPENNYKTTPPVL DSDGSFFLYSKLTVDKSRWQQGNVFSCSVMHEALHNHYTQKSLSLSPG |
| 70 | C-terminal epitope of CD16A | SFFPPGYQ |
| 71 | human CD16A | MWQLLLPTALLLLVSAGMRTEDLPKAVVFLEPQWYRVLEKDSVTLKCQGAYSPEDNSTQWF HNESLISSQASSYFIDAATVDDSGEYRCQTNLSTLSDPVQLEVHIGWLLLQAPRWVFKEED PIHLRCHSWKNTALHKVTYLQNGKGRKYFHHNSDFYIPKATLKDSGSYFCRGLFGSKNVSS ETVNITITQGLAVSTISSFFPPGYQVSFCLVMVLLFAVDTGLYFSVKTNIRSSTRDWKDHK FKWRKDPQDK |
| 72 | Linker | GGGS |
| 73 | Hexahistidin e tag | HHHHHH |

## Claims

1. A bispecific antibody construct and/or a VEGFR-2 inhibitor and/or an immune checkpoint inhibitor for use in a method of treating a tumor in a subject, wherein the bispecific antibody construct comprises at least one first binding domain capable of specifically binding CD16A and at least one second binding domain capable of specifically binding EGFR, wherein the method comprises administering to the subject the bispecific antibody construct, the VEGFR-2 inhibitor, and the immune checkpoint inhibitor.

2. The bispecific antibody construct and/or the VEGFR-2 inhibitor and/or the immune checkpoint inhibitor according to claim 1, wherein the tumor is a solid tumor; and/or wherein the tumor is an EGFR-positive tumor.

3. The bispecific antibody construct and/or the VEGFR-2 inhibitor and/or the immune checkpoint inhibitor according to claim 1 or 2, wherein the tumor is selected from the group consisting of lung cancer, colorectal cancer, liver cancer, brain cancer, kidney/renal cancer, ovarian cancer, breast cancer, squamous cell carcinoma, bladder cancer, head and neck cancer, gastric cancer, esophagus cancer, sarcoma, mesothelioma, and adenocarcinoma, optionally, non-small cell lung cancer (NSCLC), hepatocellular carcinoma (HCC), glioblastoma (GBM), Clear Cell Renal Cell Carcinoma (ccRCC), Triple-negative breast cancer (TNBC), squamous cervical cancer, and squamous cell carcinoma of head and neck (SCCHN), preferably wherein the tumor is lung cancer, optionally wherein the tumor is non-small cell lung cancer (NSCLC).

4. The bispecific antibody construct and/or the VEGFR-2 inhibitor and/or the immune checkpoint inhibitor according to any one of the foregoing claims, wherein the first binding domain binds to an epitope on CD16A which is C-terminal to the physiological Fcy receptor binding domain, said epitope preferably comprising Y158 of SEQ ID NO: 1.

5. The bispecific antibody construct and/or the VEGFR-2 inhibitor and/or the immune checkpoint inhibitor according to any one of the foregoing claims, wherein the first binding domain comprises
(i) a VH region comprising CDR-H1 sequence GYTFTSYY (SEQ ID NO: 9), CDR-H2 sequence INPSGGST (SEQ ID NO: 10), and CDR-H3 sequence ARGSAYYYDFADY (SEQ ID NO: 11), and a VL region comprising CDR-L1 sequence NIGSKN (SEQ ID NO: 12), CDR-L2 sequence QDN, and CDR-L3 sequence QVWDNYSVL (SEQ ID NO: 13);
(ii) a VH region comprising CDR-H1 sequence GYTFTSYY (SEQ ID NO: 17), CDR-H2 sequence IEPMYGST (SEQ ID NO: 18), and CDR-H3 sequence ARGSAYYYDFADY (SEQ ID NO: 19), and a VL region comprising CDR-L1 sequence NIGSKN (SEQ ID NO: 20), CDR-L2 sequence QDN, and CDR-L3 sequence QVWDNYSVL (SEQ ID NO: 21);
(iii) a VH region comprising CDR-H1 sequence GYTFTNYY (SEQ ID NO: 25), CDR-H2 sequence INPSGGVT (SEQ ID NO: 26), and CDR-H3 sequence ARGSAYYYDFADY (SEQ ID NO: 27), and a VL region comprising CDR-L1 sequence NIGSKS (SEQ ID NO: 28), CDR-L2 sequence QDK, and CDR-L3 sequence QVWDDYIVL (SEQ ID NO: 29);
(iv) a VH region comprising CDR-H1 sequence SYYMH (SEQ ID NO: 32), CDR-H2 sequence AIEPRYGSTSYAQKFQG (SEQ ID NO: 33), and CDR-H3 sequence GSAYYYDFADY (SEQ ID NO: 34), and a VL region comprising CDR-L1 sequence GGHNIGSKNVH (SEQ ID NO: 35), CDR-L2 sequence QDNKRPS (SEQ ID NO: 36), and CDR-L3 sequence QVWDNYNVL (SEQ ID NO: 37); or
(v) a VH region comprising CDR-H1 sequence NYYMQ (SEQ ID NO: 38), CDR-H2 sequence IINPSGGVTSYAQKFQG (SEQ ID NO: 39), and CDR-H3 sequence GSAYYYDFADY (SEQ ID NO: 40), and a VL region comprising CDR-L1 sequence GGNNIGSKSVH (SEQ ID NO: 41), CDR-L2 sequence QDKKRPS (SEQ ID NO: 42), and a CDR-L3 sequence QVWDDYIVL (SEQ ID NO: 43);
or wherein the first binding domain comprises
(i) a VH region having at least 80%, at least 85%, at least 90%, at least 95%, at least 98%, or at least 99% sequence identity to SEQ ID NO: 14 and a VL region having at least 80%, at least 85%, at least 90%, at least 95%, at least 98%, or at least 99% sequence identity to SEQ ID NO: 15;
(ii) a VH region having at least 80%, at least 85%, at least 90%, at least 95%, at least 98%, or at least 99% sequence identity to SEQ ID NO: 22 and a VL region having at least 80%, at least 85%, at least 90%, at least 95%, at least 98%, or at least 99% sequence identity to SEQ ID NO: 23;
(iii) a VH region having at least 80%, at least 85%, at least 90%, at least 95%, at least 98%, or at least 99% sequence identity to SEQ ID NO: 30 and a VL region having at least 80%, at least 85%, at least 90%, at least 95%, at least 98%, or at least 99% sequence identity to SEQ ID NO: 31;
(iv) a VH region having at least 80%, at least 85%, at least 90%, at least 95%, at least 98%, or at least 99% sequence identity to SEQ ID NO: 44 and a VL region having at least 80%, at least 85%, at least 90%, at least 95%, at least 98%, or at least 99% sequence identity to SEQ ID NO: 45;
(v) a VH region having at least 80%, at least 85%, at least 90%, at least 95%, at least 98%, or at least 99% sequence identity to SEQ ID NO: 46 and a VL region having at least 80%, at least 85%, at least 90%, at least 95%, at least 98%, or at least 99% sequence identity to SEQ ID NO: 47;
(vi) a VH region having at least 80%, at least 85%, at least 90%, at least 95%, at least 98%, or at least 99% sequence identity to SEQ ID NO: 48 and a VL region having at least 80%, at least 85%, at least 90%, at least 95%, at least 98%, or at least 99% sequence identity to SEQ ID NO: 49;
(vii) a VH region having at least 80%, at least 85%, at least 90%, at least 95%, at least 98%, or at least 99% sequence identity to SEQ ID NO: 50 and a VL region having at least 80%, at least 85%, at least 90%, at least 95%, at least 98%, or at least 99% sequence identity to SEQ ID NO: 51;
(viii) a VH region having at least 80%, at least 85%, at least 90%, at least 95%, at least 98%, or at least 99% sequence identity to SEQ ID NO: 52 and a VL region having at least 80%, at least 85%, at least 90%, at least 95%, at least 98%, or at least 99% sequence identity to SEQ ID NO: 53; or
(ix) a VH region having at least 80%, at least 85%, at least 90%, at least 95%, at least 98%, or at least 99% sequence identity to SEQ ID NO: 54 and a VL region having at least 80%, at least 85%, at least 90%, at least 95%, at least 98%, or at least 99% sequence identity to SEQ ID NO: 55;
or wherein the first binding domain comprises
(i) a VH region as depicted in SEQ ID NO: 14 and a VL region as depicted in SEQ ID NO: 15;
(ii) a VH region as depicted in SEQ ID NO: 22 and a VL region as depicted in SEQ ID NO: 23;
(iii) a VH region as depicted in SEQ ID NO: 30 and a VL region as depicted in SEQ ID NO: 31;
(iv) a VH region as depicted in SEQ ID NO: 44 and a VL region as depicted in SEQ ID NO: 45;
(v) a VH region as depicted in SEQ ID NO: 46 and a VL region as depicted in SEQ ID NO: 47;
(vi) a VH region as depicted in SEQ ID NO: 48 and a VL region as depicted in SEQ ID NO: 49;
(vii) a VH region as depicted in SEQ ID NO: 53 and a VL region as depicted in SEQ ID NO: 50;
(viii) a VH region as depicted in SEQ ID NO: 55 and a VL region as depicted in SEQ ID NO: 51; or
(ix) a VH region as depicted in SEQ ID NO: 52 and a VL region as depicted in SEQ ID NO: 53;
optionally wherein the first binding domain is a variable domain (Fv), a single chain Fv (scFv), a Fab, a single chain diabody (scDb), a diabody (Db) or a double Fab.

6. The bispecific antibody construct and/or the VEGFR-2 inhibitor and/or the immune checkpoint inhibitor according to any one of the foregoing claims, wherein the second binding domain comprises a VH and a VL domain of an antibody;
optionally
wherein the second binding domain comprises a VH region comprising CDR-H1 sequence GGSVSSGSYY (SEQ ID NO: 56), CDR-H2 sequence IYYSGST (SEQ ID NO: 57), and CDR-H3 sequence ARNPISIPAFDI (SEQ ID NO: 58), and a VL region comprising CDR-L1 sequence NIGSKS (SEQ ID NO: 59), CDR-L2 sequence YDS, and CDR-L3 sequence QVWDTSSDHVL (SEQ ID NO: 60),
or wherein the second binding domain comprises a VH region having at least 80%, at least 85%, at least 90%, at least 95%, at least 98%, or at least 99% sequence identity to SEQ ID NO: 61 and a VL region having at least 80%, at least 85%, at least 90%, at least 95%, at least 98%, or at least 99% sequence identity to SEQ ID NO: 62, preferably wherein the second binding domain comprises a VH region as depicted in SEQ ID NO: 61 and a VL region as depicted in SEQ ID NO: 62;
wherein the second binding domain is optionally a variable domain (Fv), a single chain Fv (scFv), a Fab, a single chain diabody (scDb), a diabody (Db) or a double Fab.

7. The bispecific antibody construct and/or the VEGFR-2 inhibitor and/or the immune checkpoint inhibitor according to any one of the foregoing claims, wherein the bispecific antibody construct binds to a target cell and an immune effector cell simultaneously, optionally wherein the bispecific antibody construct is AFM24.

8. The bispecific antibody construct and/or the VEGFR-2 inhibitor and/or the immune checkpoint inhibitor according to any one of the foregoing claims, wherein the VEGFR-2 inhibitor is an anti-VEGFR-2 antibody; optionally wherein the VEGFR-2 inhibitor is selected from the group consisting of ramucirumab, bevacizumab, aflibercept; preferably wherein the VEGFR-2 inhibitor is ramucirumab.

9. The bispecific antibody construct and/or the VEGFR-2 inhibitor and/or the immune checkpoint inhibitor according to any one of the foregoing claims, wherein the immune checkpoint inhibitor is a PD-L1 or PD-1 inhibitor; optionally wherein the immune checkpoint inhibitor is an anti-PD-1 antibody or an anti-PD-L1 antibody; optionally wherein the immune checkpoint inhibitor is selected from the group consisting of nivolumab, atezolizumab, pembrolizumab cemiplimab, H4H7798N, dostarlimab, durvalumab, pidilizumab, MED10608, BI 754091, PF-0680159, spartalizumab, camrelizumab, JNJ-63723283, MCLA-134, H2M8314N, avelumab, MDX-1105, LY3300054, FAZ053, STI-1014, CX-072, KN035, and CK-301, preferably wherein the immune checkpoint inhibitor is nivolumab, atezolizumab or pembrolizumab, more preferably wherein the checkpoint inhibitor is atezolizumab..

10. The bispecific antibody construct and/or the VEGFR-2 inhibitor and/or the immune checkpoint inhibitor according to any one of the foregoing claims, wherein the bispecific antibody construct is administered to the subject once every about 6 to about 8 days, or once every about 7 days; and/or wherein the VEGFR-2 inhibitor is administered to the subject once every about 1 to about 3 weeks, once every about 2 weeks, once every about 12 to about 16 days, once every about 13 to about 15 days, or once every about 14 days; and/or wherein the immune checkpoint inhibitor is administered to the subject once every about 1 to about 3 weeks, once every about 2 weeks, once every about 12 to about 16 days, once every about 13 to about 15 days, or once every about 14 days;
optionally wherein the bispecific antibody construct is administered at a dose in the range of about about 300 to about 800 mg, about 400 to about 550 mg, about 420 to about 530 mg, about 450 to about 500 mg, or about 480 m; and/or wherein the VEGFR-2 inhibitor is administered at a dose in the range of about 5 to about 15 mg, about 8 to about 12 mg, about 9 to about 11 mg, or about 10 mg, and/or wherein the immune checkpoint inhibitor is administered at a dose in the range of about 500 to about 1200 mg, about 700 to about 1000 mg, about 800 to about 900 mg, or about 840 mg.

11. The bispecific antibody construct and/or the VEGFR-2 inhibitor and/or the immune checkpoint inhibitor according to any one of the foregoing claims, wherein treating results in about 5% (size) or greater shrinkage of tumor, about 10% or greater shrinkage of tumor, about 20% or greater shrinkage of tumor, about 30% or greater shrinkage of tumor, or about 50% or greater shrinkage of tumor;
and/or wherein treating reduces the number of regulatory T cells in the tumor microenvironment as compared to a treatment regimen comprising the administration of the bispecific antibody molecule and the immune checkpoint inhibitor, but without the administration of a VEGFR-2 inhibitor;
and/or wherein treating inhibits, reduces, or reverts the transformation of a tumor microenvironment to an immune-suppressive tumor-microenvironment as compared to a treatment regimen comprising the administration of the bispecific antibody molecule and the immune checkpoint inhibitor, but without the administration of a VEGFR-2 inhibitor;
and/or wherein treating inhibits, reduces, or reverts the transformation of a tumor microenvironment to an immune-suppressive tumor-microenvironment as compared to a treatment regimen comprising the administration of the bispecific antibody molecule and the immune checkpoint inhibitor, but without the administration of a VEGFR-2 inhibitor

12. A composition comprising a VEGFR-2 inhibitor and a bispecific antibody construct that comprises at least one first binding domain capable of specifically binding CD16A and at least one second binding domain capable of specifically binding EGFR.

13. A combination comprising a VEGFR-2 inhibitor, a bispecific antibody construct that comprises at least one first binding domain capable of specifically binding CD16A and at least one second binding domain capable of specifically binding EGFR, and optionally an immune checkpoint inhibitor.

14. A VEGFR-2 inhibitor or a combination of a VEGFR-2 inhibitor, a bispecific antibody construct and optionally an immune checkpoint inhibitor, wherein the bispecific antibody construct comprises at least one first binding domain capable of specifically binding CD16A and at least one second binding domain capable of specifically binding EGFR,
for use in a method of inhibiting, reducing, or reverting the increase of regulatory T cells in a tumor microenvironment associated with the treatment of a tumor with a bispecific antibody construct and optionally an immune checkpoint inhibitor;
or
for use in a method of inhibiting, reducing, or reverting the transformation of a tumor microenvironment to an immune-suppressive tumor-microenvironment associated with the treatment of a tumor with a bispecific antibody construct and optionally an immune checkpoint inhibitor;
or
for use in a method of inhibiting, reducing, or reverting neovascularization associated with the treatment of a tumor with a bispecific antibody construct and optionally an immune checkpoint inhibitor.

15. The composition according to claim 12, the combination according to claim 13, or the VEGFR-2 inhibitor or a combination of a VEGFR-2 inhibitor, a bispecific antibody construct and optionally an immune checkpoint inhibitor according to claim 14 for use in a method as defined in any one of claims 1 to 11.
